(19) Europäisches Patentamt
European Patent Office
Office européen des brevets

(11) Publication number: 0 010 299
B1

(12) EUROPEAN PATENT SPECIFICATION

(45) Date of publication of patent specification: 15.02.84

(21) Application number: 79104025.6

(22) Date of filing: 18.10.79

(51) Int. Cl.[3]: A 61 K 45/06,
A 61 K 31/785, A 61 K 9/18
//(A61K31/785, 31/365),
(A61K31/785, 31/235)

# (54) Pharmaceutical composition useful for the treatment of hypercholesteremia.

(30) Priority: 19.10.78 US 952864
21.08.79 US 66752

(43) Date of publication of application:
17.09.80 Bulletin 80/9

(45) Publication of the grant of the patent:
15.02.84 Bulletin 84/7

(84) Designated Contracting States:
AT BE CH DE FR GB IT LU NL SE

(56) References cited:
AU - B - 439 164
GB - A - 1 286 949
US - A - 3 692 895
US - A - 3 983 140
US - A - 4 098 726

The file contains technical information submitted after the application was filed and not included in this specification

(73) Proprietor: MERCK & CO. INC.
126, East Lincoln Avenue P.O. Box 2000
Rahway New Jersey 07065 (US)

(72) Inventor: Kuron, Gunther W.
94 Juniper Drive
Freehold, N.J. 07728 (US)

(74) Representative: Abitz, Walter, Dr.-Ing. et al,
Abitz, Morf, Gritschneder P.O. Box 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 010 299 B1

Courier Press, Leamington Spa, England.

# Pharmaceutical composition useful for the treatment of hypercholesteremia

## BACKGROUND OF THE INVENTION

It has long been known that certain diseases, such as coronary heart disease and atherosclerosis, may be caused by the presence of too high a cholesterol level in the blood plasma and there have been many attempts to provide a medicament formulation for oral administration to reduce this cholesterol level.

A number of pharmacological mechanisms have been postulated as usable to reduce cholesterol in the body. The binding of cholesterol itself into a non-depositable form by e.g. phytosterols has been tried. Certain phenoxyalkanoic acids — e.g. chlorphenoxy-isobutyric acid (CPIB) and its esters, or halofenate — reduce the high density lipoproteins in which cholesterol is bound in rats and thus reduce blood cholesterol in that species, but are not particularly effective in humans who have the cholesterol associated with low density lipoproteins.

Attempts have also been made to block the synthesis of cholesterol. The rate limiting step in cholesterol biosynthesis is known to the action of the enzyme 3-hydroxy-3-methylglutaryl-CoA reductase (hereinafter HMG—CoA reductase). A number of compounds have been reported to inhibit this enzyme but their activity for the most part have been too weak to show much effect.

Recently, Endo et al., *Antibiotics,* 1346—48, *29* (1976), have reported that 1, 2, 3, 7, 8, 8a-hexahydro-7-methyl-8-[2-(3,4,5,6-tetrahydro-4-hydroxy-6-oxo-2H-pyran-2-yl]-ethyl)-1-naphthyl 2-methyl butanoate, (known as ML 236B), reduced serum cholesterol levels in the acutely treated rat. The same group of workers have shown, *Eur. J. Bio. Chem. 77* (1977), that this compound is a very potent inhibitor of the reductase enzyme. In fact, the $ED_{50}$ in the inhibition test has been found to be of the order of $10^{-8}$. This was the first inhibitor of such potency to be reported. However, Bensch et al., *Biochemical and Biophysical Research Communications,* 247—254, *82* (1978) report that they had found no effect on serum cholesterol levels compared with fed or fasted controls. Furthermore, Bensch et al., observed no change in either liver or liver microsomal cholesterol concentration. Bensch et al., demonstrated a marked increase in HMG—CoA reductase, as observed in fed rats three hours following the administration of ML—236B. The increase HMG—CoA reductase activity found in rats and fibroblasts treated with 1,2,3,7,8,8a-hexahydro-7-methyl-8-[2-(3,4,5,6-tetrahydro-4-hydroxy-6-oxo-2H-pyran-2-yl]ethyl)-1-naphthyl 2-methyl butanoate suggests that the production of HMG—CoA reductase may be induced and, therefore produces an increase in enzyme protein synthesis. The induction of this enzyme indicates that as the number of active molecules of HMG—CoA reductase was reduced by the competitive inhibitor, 1,2,3,7,8,8a-hexahydro-7-methyl-8-[2-(3,4,5,6-tetrahydro-4-hydroxy-6-oxo-2H-pyran-2-yl]ethyl)-1-naphthyl 2-methyl butanoate, the cell responds by increased synthesis of the enzyme protein in order to maintain a basic rate of cholesterol biosynthesis.

More recently, Monaghan et al., have described the isolation from fermentation related compounds, e.g. MSD 803, described below, which are even more potent inhibitors of the enzyme and Willard et al., have described related synthetic compounds which also are potent inhibitors of the enzyme. These, together with the Endo et al., compounds form a unique class of HMG—CoA reductase inhibitors with $ED_{50}$ of less than $10^{-6}$. Both these compounds are described below.

Still another pharmacological method which has been tried for the lowering of blood cholesterol is oral ingestion of bile acid sequestrants-compounds, usually cationic polymers, which will bind the bile acids into a non-reabsorbable form in the gastronintestinal tract, thus forcing the liver to extract cholesterol from the blood for oxidation to bile acids. This often results in increased synthesis of cholesterol to compensate.

None of these methods have resulted in really significantly major reductions in blood cholesterol, sufficient to afford an effective atherosclerosis treatment. Some attempts have been made to combine various agents. Various bile acid sequestrants of different chemical structure have been reported. Coadministration of CPIB with a bile acid sequestrant has resulted in some increase in the lowering (Howard, U.S. patent 3,846,541) but the results of these experiments were cholesterol level reductions insufficient to be an effective treatment of hypercholesteremia.

## SUMMARY OF THE INVENTION

I have found that the co-administration of certain specific synthesis inhibitors with certain bile acid sequestrants, as defined below, results in a major reduction in cholesterol levels, a reduction above and beyond what one would expect from the addition of the effects of one treatment to the effects of the other which is of such magnitude (up to 80%) as to make this an effective treatment of hypercholesteremia. A treatment which lowers cholesterol levels by 30% does not bring most patients down to a normal cholesterol level. A reduction of 80% represents a treatment which is useful for even the most severe cases of hypercholesteremia.

The synthesis inhibitors are those which operate at the HMG—CoA reductase level with an $ED_{50}$ in one or both of the standard tests less than $10^{-6}$. The inhibitory activity of these compounds and the biosynthesis of cholesterol has been measured by two methods. The experimental method A has been realized *in vitro* by the method of H. J. Knauss et al., J. Biol. Chem., *234*, 2835, (1959) and the activity was expressed as the molar concentration $I_{50}(M)$ necessary for the inhibition of 50% of the enzymatic

activity. The experimental method B was the method of A. A. Kandutsch, et al., J. Biol. Cheml, *248* 8403 (1973) for measuring the quantity of $C^{14}$ cholesterol biosynthesis from acetic acid $c^{14}$ in mouse L cells. The activity is expressed as the molar concentration $I_{50}$(M) necessary for inhibition of 50% of the biosynthesis of cholesterol. The synthesis inhibitors which meet the criterion of $ED_{50}$ less than $10^{-6}$ comprise those described respectively by Endo et al., those described by Monaghan et al., and those described by Willard et al., cited above, of which the preferred species is one of those described by Monaghan.

The bile acid sequestrants used in the compositions of this invention are those pharmaceutically acceptable cationic polymers, described below, which bind bile acids in the gastrointestinal tract so that they cannot be reabsorbed by the intestines and are excreted in the faeces. Of those described, the greatly preferred species is cholestyramine, a quarternized polystyrene polyvinyl benzene copolymer.

DESCRIPTION OF THE INVENTION

This invention relates to pharmaceutical compositions having marked hypocholesteremic activity. The compositions of the present invention have been found to act synergistically in producing a marked reduction in the cholesterol level in plasma. The compositions comprise two components and the treatment involves the co-administration of these components. Component A is a cholesterol synthesis inhibitor, described below, acting at the HMG—CoA reductase level with an $ED_{50}$ in the inhibition test using the method described by Beg, Stonik, Brewer and Bryan (1977 *FEBS Letters 80* 123 to 129) using enzymes prepared as described by Kleinsek, Rangatham and Porter (1977 *Proc. Nat. Acad. Sci. 74* 1431 to 1435), of no more than $10^{-6}$. Component B is a polymeric anion exchange resin, described below.

*A. The Cholesterol Synthesis Inhibitors*

1. *Compactin* (ML 236 B)

Endo et al., have described (U.S. 3,983,140, issued September 28, 1976 and West German Offenlegungschrift 2,748,825, compounds of the formula I and the corresponding open hydroxy acids of formula I'

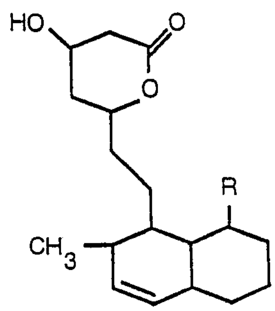

(I)

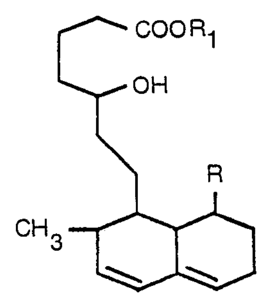

(I')

wherein

R is hydrogen atom, hydroxy group or the 2-methylbutyryloxy group [—$OCOCH(CH_3)CH_2CH_3$] and $R_1$ is hydrogen, alkyl; or phenacyl or benzyl both optionally substituted by alkyl, alkoxy or halogen; the group 1/n M (where M is a metal and n is its valency) or the group $AH^+$ where A is an amino acid wherein alkyl contains 1 to 6 carbon atoms. The preferred compound of this type is the case wherein R in formula (I) above is [—$OCOCH(CH_3)CH_2CH_3$] having the chemical designation: 1,2,3,7,8,8a-hexahydro-7-methyl-8-[2-(3,4,5,6-tetrahydro-4-hydroxy-6-oxo-2H-pyran-2-yl]ethyl)-1-naphthyl 2-methyl butanoate, and the structural formula (II) or the corresponding open lactone ring compounds having the general formula (II'):

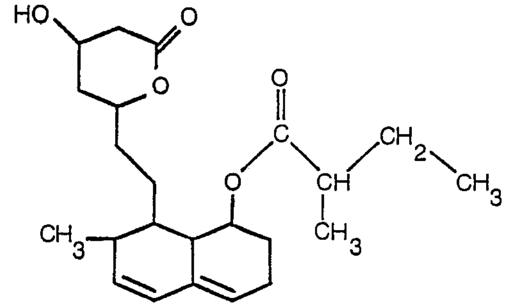

(II)

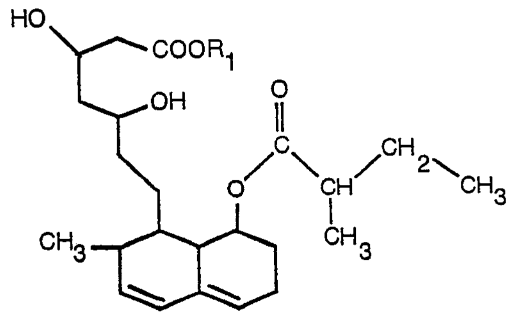

(II')

wherein $R_1$ is as described above. These products are isolated from a fermentation of amicroorganism of the genus Penicillum, as described in said Endo et al., patent. The compound II has been given the name compactin.

2. *MSD 803*

Another group of related compounds with even more potent activity of the same type have been described by Monaghan, Albert Hoffman and Albers-Schöberg.

They are products from the cultivation of a microfungus of the species *Aspergillus* and have been identified as compounds of the formula:

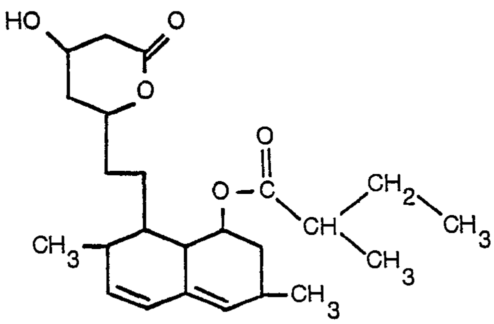

III

or its corresponding free hydroxyacid form

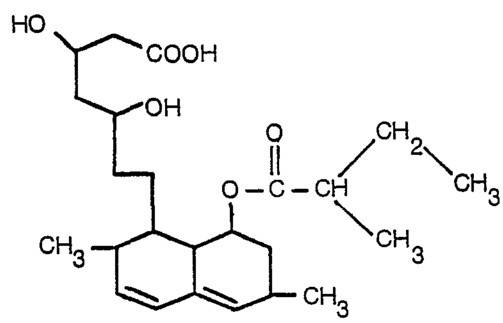

III'

as well as pharmaceutically acceptable salts of the latter and lower alkyl and substituted alkyl esters of the latter in which the possible substituent is phenyl, dimethylamino or acetylamino. The lactone of the above structure, or its free hydroxy acid form of the above structure, has been designated MSD 803. Although closely related to the compactin of Endo et al., the MSD 803, of Monaghan et al., is a much more active compound and is the preferred species of cholesterol synthesis inhibitor in the compositions of this invention. The following description is extracted from said Monaghan et al., application.

The compounds are prepared by cultivating a microorganism belonging to the genus *Aspergillus* and then recovering said compounds of this invention from the cultured broth. Based upon taxonomic studies, this Aspergillus, isolated and identified as a hitherto undescribed microorganism, has been designated MF—4833 in the culture collection of Merck and Co., Inc. Rahway, NJ and a culture thereof has been placed on permanent deposit with the American Type Culture Collection 12301 Parklawn Drive, Rockville, Maryland 20852, and has been assigned accession No. ATCC No. 20541 (deposition date May 31, 1979). Another sample, of a similar organism, designated MF—4845 in the Merck culture collection, has likewise been placed on deposit with the American Type Culture Collection, 12301 Parklawn Drive, Rockville, Maryland 20852 and has been given the accession number ATCC 20542, (deposition date May 31, 1979). The latter organism is the one giving the better yield. Other

organisms of the genus *Aspergillus*, including mutants of the above ones are also capable of producing MSD803.

The morphological characteristics of the microorganisms MF—4833 and MF—4845 have been found to be those of the genus *Aspergillus*. Using the criteria specified in the standard authority "Manual of the Aspergilli", Charles Thom and Kenneth B. Rasper, published by the Williams and Wilkins Company, Baltimore, Md., 1945, and by comparison with known species, it has been determined that both strains are *Aspergillus terreus*.

The culture of these organisms to produce MSD803 is carried out in aqueous media such as those employed for the production of other fermentation products. Such media contain sources of carbon, nitrogen and inorganic salts assimilable by the microorganism.

In general, carbohydrates such as sugars, for example, glucose, fructose, maltose, sucrose, xylose, mannitol and the like and starches such as grains, for example, oats, ryes, cornstarch, corn meal and the like can be used either alone or in combination as sources of assimilable carbon in the nutrient medium. The exact quantity of the carbohydrate source of sources utilized in the medium depend in part upon the other ingredients of the medium but, in general, the amount of carbohydrate usually varies between about 1% and 6% by weight of the medium. These carbon sources can be used individually, or several such carbon sources may be combined in the medium. In general, many proteinaceous materials may be used as nitrogen sources in the fermentation process. Suitable nitrogen sources include for example, yeast hydrolysates, primary yeast, soybean meal, cottonseed flour, hydrolysates of casein, corn steep liquor, distiller's solubles or tomato paste and the like. The sources of nitrogen either alone or in combination, are used in amounts ranging from about 0.2% to 6% by weight of the aqueous medium.

Among the nutrient inorganic salts which can be incorporated in the culture media are the customary salts capable of yielding sodium, potassium, ammonium, calcium, phosphate, sulfate, chloride, carbonate, and like ions. Also included are trace metals such as cobalt, manganese, iron and magnesium.

It should be noted that the media described in the detailed procedure below are merely illustrative of the wide variety of media which may be employed, and are not intended to be limitative. Specifically, the carbon sources used in the culture media to produce MSD803 included dextrose, dextrin, oat flour, oatmeal, molasses, citrate, soybean oil, glycerol, malt extract, cod liver oil, starch, ethanol, figs, sodium ascorbate and lard oil. Included as nitrogen sources were peptonized milk, autolyzed yeast, yeast RNA, tomato paste, casein, primary yeast, peanut meal, distillers solubles, corn steep liquor, soybean meal, corn meal, NZ amine, beef extract, aspargine, cottonseed meal and ammonium sulfate. The major ionic components were $CaCO_3$, $KH_2PO_4$, $MgSO_4 \cdot 7H_2O$ and NaCl and small amounts of $CoCl_2 \cdot 6H_2O$ and traces of Fe, Mn, Mo, B and Cu were also present.

The fermentation is carried out at temperatures ranging from about 20° to 37°C.; however, for optimum results it is preferable to conduct the fermentation at temperatures of from about 22° to 30°C. The pH of the nutrient media suitable for growing the *Aspergillus* culture and producing MSD803 can vary from about 6.0 to 8.0.

Although the compound is produced by both surface and submerged culture, it is preferred to carry out the fermentation in the submerged state. A small scale fermentation is conveniently carried out by inoculating a suitable nutrient medium with the *Aspergillus* culture and, after transfer to a production medium, permitting the fermentation to proceed at a constant temperature of about 28°C. on a shaker for several days.

The fermentation is initiated in a sterilized flask of medium via one or more stages of seed development. The nutrient medium for the seed stage may be any suitable combination of carbon and nitrogen sources. The seed flask is shaken in a constant temperature chamber at about 28°C. for 2 days, or until growth is satisfactory, and some of the resulting growth is used to inoculate either a second stage seed or the production medium. Intermediate stage seed flasks, when used, are developed in essentially the same manner, that is, part of the contents of the flask from the last seed stage are used to inoculate the production medium. The inoculated flasks are shaken at a constant temperature for several days, and at the end of the incubation period the contents of the flasks are centrifuged or filtered.

For large scale work, it is preferable to conduct the fermentation in suitable tanks provided with an agitator and a means of aerating the fermentation medium. According to this method, the nutrient medium is made up in the tank and sterilized by heating at temperatures of up to about 120°C. Upon cooling, the sterilized medium is inoculated with a previously grown seed of the producing culture, and the fermentation is permitted to proceed for a period of time as, for example, from 3 to 5 days while agitating and/or aerating the nutrient medium and maintaining the temperature at about 28°C. This method of producing MSD803 is particularly suited for the preparation of large quantities.

The compound is conveniently isolated from the fermentation broth as the lactone. However, MSD803 is present in the fermentation broth largely as the hydroxycarboxylate (open lactone) form. It is possible to isolate this form and its salts. Alternatively, the lactone form can be hydrolyzed with bases such as NaOH to yield the corresponding salts such as the sodium salts. The use of bases with the pharmaceutically acceptable cations affords salts of these cations. Careful acidification of the salts affords the hydroxy acid form. Conversely, the hydroxy acid can be converted to the lactone form at

acidic pH. Opening the lactone, under catalysis, with methanol, ethanol, propanol, or butanol or with phenyl, dimethylamino, or acetylamino alkanols yields the corresponding esters of this invention.

The physico-chemical properties of MSD803 in its lactone form are summarized as follows:

| | |
|---|---|
| 1. *Melting point* | 170—171° |
| 2. *Molecular Weight* (mass spectrum) | 404 |
| 3. *Formula* (found by mass spectrometry calculated) | $C_{24}H_{36}O_5$ 404.2555 404.2563 |
| 4. *UV Spectrum* (in acetonitrile): | *Maxima* 230.5 nm with E% 505.7 237.5 nm with E% 576.6 246 nm with E% 395.2 |

5. *$^{13}C$ NMR chemical shifts.* The spectrum has been recorded in $CDCl_3$ solution (20.1 mg in 0.35 ml). Chemical shifts are given relative to internal tetramethylsilane at zero ppm; under the experimental conditions the solvent ($CDCl_3$) signal appears centered at 70.0 ppm. In agreement with mass spectral data 24 carbon atoms are observed; their chemical shifts are:

   11.5, 13.6, 16.0, 22.6, 24.1, 26.6, 27.2, 30.5, 32.8, 35.9, 36.4, 37.1, 38.4, 41.3, 62.4, 67.8, 76.4, 128.4, 129.7, 131.7, 133.2, 170.8 and 177.2 ppm.

6. *Optical rotation.*
   The specific optical rotation $[\alpha]_D^{25} = 320.7°$ has been determined on a solution of 5.30 mg/ml $CH_3CN$. This value has been obtained by measuring at the sodium-D-line wave length.

On the basis of these and other data such as NMR and IR spectra, the structure of the product believed with a considerable degree of certainty to have the chemical structure (III) above;

The preparation of MSD 803 and its salts and esters can be illustrated by the following:

*Fermentation*

The medium used in each step of the fermentation comprised:

| | |
|---|---|
| Corn steep liquor | 5 g |
| Tomato paste | 40 g |
| Oat Flour | 10 g |
| Glucose | 10 g |
| Trace element solution | 10 ml |
| Distilled water | 1000 ml |

adjusted to pH 6.8 with sodium hydroxide.

The trace element solution comprised:

| | |
|---|---|
| $FeSO_4 \cdot 7H_2O$ | 1 g |
| $MnSO_4 \cdot 4H_2O$ | 1 g |
| $CuCl_2 \cdot 2H_2O$ | 25 mg |
| $CaCl_2$ | 100 mg |
| $H_3BO_4$ | 56 mg |
| $(NH_4)_6 Mo_7O_{24} \cdot 4H_2O$ | 19 mg |
| $Zn SO_4 \cdot 7H_2O$ | 200 mg |
| distilled water | 1 liter |

All mediums were checked for sterility before innoculation with a microorganism.

To a 250 ml non-baffled Erlenmeyer flask was charged 40 ml of medium and the contents of one tube of lyophillized organism MF 4833. It was then shaken for 24 hours at 28°C on a rotary shaker at 220 rpm. New flasks were then charged with 40 ml of medium and 1 ml of the first flasks' contents and were shaken an additional 24 hours at 28°C. A 2 liter flask was then charged with 400 ml of medium and 10 ml of the second stage fermentation mixture and this too was shaken for 24 hours at 28°C.

A 200 gallon stainless steel fermentation vat was then charged with 501 liters of a medium comprising:

| | |
|---|---|
| lactose | 2% wt/vol |
| distiller solubles | 1.5% wt/vol |
| autolyzed yeast | 0.5% wt/vol |
| Polyglycol P2000 | 0.25% wt/vol |

whose pH was adjusted to 7.0. This was sterilized 15 minutes at 121°C. One liter of the third stage above was then charged and the mixture was incubated at 130 rpm at 28°C for 96 hours with an air flow of 10 cfm.

B. *Isolation of MSD803*

About 37.5 lbs. (3/4 bag) of a silicaceous filter aid was added to 110 gal. whole broth from the culture of MF—4833 described above and the mixture was filtered through an 18-inch filter press. The clarified filtrate, (pH 6.6) was adjusted to pH 4.0 by careful addition of 450 ml of concentrated hydrochloric acid, and extracted by agitation with about one-third volume (36 gal.) of ethyl acetate. After separation, the upper solvent layer was removed, and the water phase again extracted with ethyl acetate (38 gal.) in a similar fashion. After separation, the two extracts were combined and backwashed by agitation with about twelve gallons of water. After separation, the ethyl acetate solution was concentrated under vacuum at a temperature below 30°C, first in a stirred kettle, and finally in a rotary vacuum evaporator to a residual volume of slightly less than one gallon.

Approximately 1 gal. (3800 ml) of ethyl acetate concentrate from the preceding extraction was further concentrated in a rotary evaporator (*ca* 10 mm, 40°C bath) to a syrup and was then concentrated twice more, after addition of about one liter of methylene chloride in two portions, to free the syrup of polar solvent. The final oil of about 300 ml which contained about 250 g of solids by dry weight determination, was made up to about 750 ml with ethyl acetate methylene chloride (30/70; v/v) and 200 g of silica gel was added and mixed in to form a slurry. This was layered over the top of a 14 cm by 36 cm column bed holding 2.5 kg of the same silica gel, in about 7.5 l volume, which had been packed as a slurry in the same solvent mixture. Development with the same solvent was continued until 3 liters of effluent was taken off as forerun.

Development with ethyl acetate-methylene chloride (50/50; v/v) was begun, taking 800 ml effluent fractions. Twelve fractions were taken, then 100% ethyl acetate elution was begun, and after seven more fractions, 100% acetone elution was begun. Fractions four through twenty-four were assayed for bio-activity in the HMG—CoA Reductase inhibition assay referred to in Example 1. Substantial activity was found in fractions 7 through 11. Peak activity was found in fraction 8. It was concentrated to an oil for further purification; dry wt. by solids determination was 9.0 gm.

Fraction 8 from the silica gel column was triturated with 50 ml methylene chloride and filtered; the dried filter cake weighed 4.9 gm. The filtrate was charged to a 2-inch I.D. by 1-meter long column filled with Sephadex LH—20 dextran gel (Pharmacia) swollen and equilibrated in methylene chloride, and the column was eluted with methylene chloride at a rate of 15 ml/min. MSD803 is eluted between 0.64 and 0.81 column volumes. Solvent was removed from this peak leaving a slightly brown residue weighing approximately 0.290 gm. 213 mg. of this residue was taken up in 1.5 ml of $CH_2Cl_2$—$CH_3CN$ (65—35), charged to a prepacked and equilibrated silica gel column (EM LOBAR Size B) and eluted with $CH_2Cl_2$—$CH_3CN$ (65—35) at 5 ml/min. Evaporation of solvent from the peak eluting between 235 and 360 ml of eluant left 121 mg of crystalline product, m.p. 155—160°C. HPLC of this material on a EM RP 18 reverse-phase analytical column (E Merck HIBAR II, Cat. No. 906046) using 0.05M sodium phosphate pH 3.0-acetonitrile 45—55 as eluant at 2 ml/min. showed a characteristic uv absorbing peak at 11 min.

Eighty-two mg of this material was recrystallized from 0.6 ml of absolute ethanol, then again from 0.4 ml of the same solvent to afford, after drying over-night in a desiccator over $P_2O_5$, 40 mg of white feathery crystals. Analytical HPLC on the system described above gave a single sharp peak at 11 minutes elution time. After further recrystallizations, a melting point of 170—171°C was obtained.

The product was identified by spectra, etc., as the lactone form of MSD803. This material, in the in vitro HMG—CoA reductase test (of Example 1) gave an $1C_{50}$ of 0.01 micrograms per milliliter.

*Preparation of Salts of MSD803*

To a solution of 40 mg the product prepared above in 2 ml of ethanol is added 1 ml of aqueous NaOH ($10^{-4}$ moles; 1 equivalent). After one hour at room temperature, the mixture is taken to dryness *in vacuo* to yield the sodium salt of the free acid form of MSD803.

In like manner the potassium salt is prepared using one equivalent of potassium hydroxide and the calcium salt using one-half equivalent of CaO. Other pharmaceutically acceptable salts are likewise prepared using equivalent quantities of the appropriate base.

*Preparation of Free Hydroxy Acid of MSD803*

The sodium salt prepared above is redissolved in 2 ml of ethanol-water (1:1) and added to 10 ml of 0.1N hydrochloric acid from which the liberated hydroxy acid is extracted with ethyl acetate. The latter solvent is washed once with water, dried and removed *in vacuo* with a bath temperature not exceeding 30°. The hydroxy acid derived slowly reverts to the lactone on standing.

*Preparation of esters of MSD803*

To a solution of 4 mg of the product prepared above in 1 ml of absolute ethanol is added 0.1 ml 0.1M sodium ethoxide in absolute ethanol. This solution is allowed to stand at room temperature for one hour, is then diluted into water and extracted twice with ethylacetate, the ethyl acetate dried over anhydrous sodium sulfate is removed *in vacuo* to yield the ethyl ester of MSD803.

In like manner, by the use of equivalent amounts of methanol, propanol, butanol, isobutanol, t-butanol, amylalcohol, isoamylalcohol, 2-dimethylaminoethanol, benzylalcohol, phenethanol, 2-acetamidoethanol and the like, the corresponding esters are obtained.

3. *(+)-trans-6-phenyl-E-4-hydroxytetrahydro-pyran-2-ones*

A third group of compounds having the same cholesterol synthesis inhibiting activity comprises the (+)-trans-6-phenyl-E-4-hydroxytetrahydro-pyran-2-ones described by Willard et al.

These are compounds having the structure

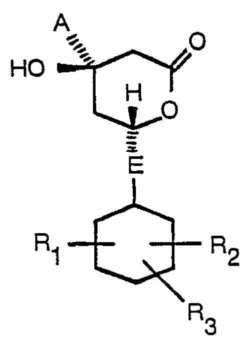

IV

wherein

A is H or methyl;

E is a direct bond, $—CH_2—$, $—CH_2—CH_2—$, $—CH_2—CH_2—CH_2$ or $—CH = CH—$;

$R_1$, $R_2$ and $R_3$ are each selected from

- H,
- halogen
- $C_{1-4}$ alkyl,
- $C_{1-4}$ haloalkyl,
- phenyl,
- phenyl substituted by
  - halogen,
  - $C_{1-4}$ alkoxy
  - $C_{2-8}$ alkanoyloxy
  - $C_{1-4}$ alkyl,

$OR_4$ in which $R_4$ is

- H,
- $C_{2-8}$ alkanoyl,
- benzoyl,
- phenyl,
- halophenyl,
- phenyl $C_{1-3}$ alkyl,

$C_{1-9}$ alkyl,
cinnamyl,
$C_{1-4}$ haloalkyl,
$C_{3-6}$ cycloalkyl-$C_{1-3}$-alkyl,
adamantyl, adamantyl-$C_{1-3}$-alkyl, or
substituted phenyl $C_{1-3}$ alkyl in each of which the substituents are selected from
halogen,
$C_{1-4}$ alkoxy,
$C_{1-4}$ alkyl, or
$C_{1-4}$ haloalkyl;

and the corresponding dihydroxy acids in which the lactone ring is opened, and the pharmaceutically acceptable salts of said acids, and the lower alkyl and phenyl, dimethylamino or acetylamino substituted lower alkyl esters of said dihydroxy acids; all said compounds being the 4-*R* enantiomer of the trans isomer whose structure is shown. An especially preferred embodiment of the compounds for use in this invention are those in which E is $—CH_2—CH_2—$, $R_1$ and $R_2$ are respectively 2-halo and 4-halo (especially chloro) and $R_6$ is halophenoxy or halophenylalkoxy (especially p-fluorobenzyloxy or p-fluorophenoxy).

These compounds are prepared as illustrated in the following Flow Sheets and other data.

FLOW SHEET 1

Synthesis of Ethylene Bridged Compounds

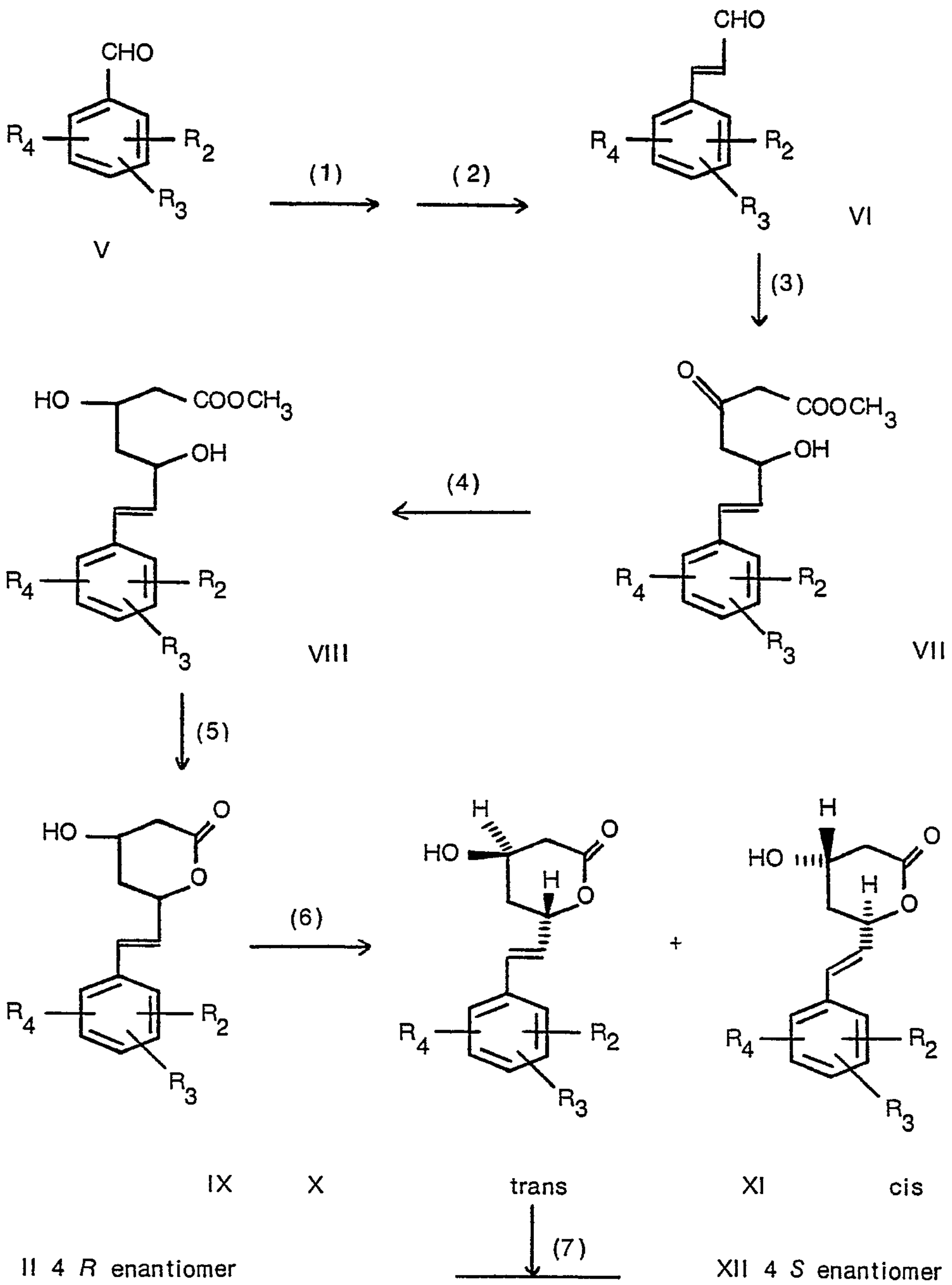


V

VI

VII

VIII

IX X trans

XI cis

II 4 *R* enantiomer

XII 4 *S* enantiomer

DEFINITIONS: $R_2$, $R_3$ and $R_4$ are defined in specifications.

## FLOW SHEET II

Preparation of Salts, Esters, Free Hydroxy acids

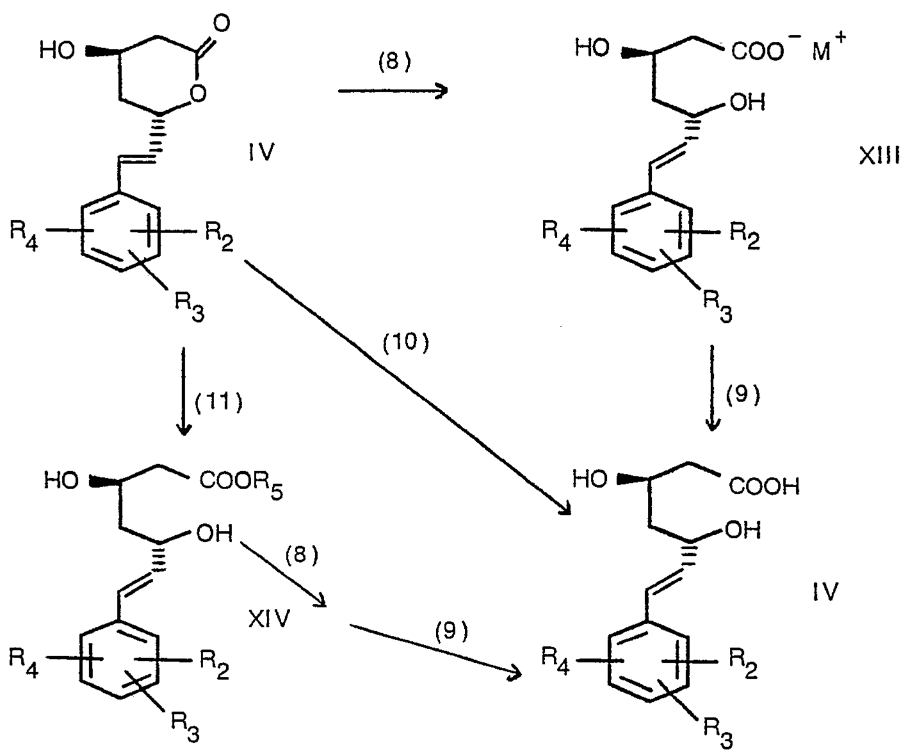


DEFINITIONS:

$R_2$, $R_3$ and $R_4$ as defined in specification.

$R_5$ = $C_{1-5}$ lower alkyl or $C_{1-5}$ lower alkyl substituted by phenyl, dimethyl amino or acetamino.

$M^+$ = pharmaceutically acceptable cation.

## FLOW SHEET III

### COMPOUNDS WITH OTHER BRIDGING GROUPS

A. Direct Bond from phenyl to lactone ring

(Procedure of Flow Sheet I omitting step (2)

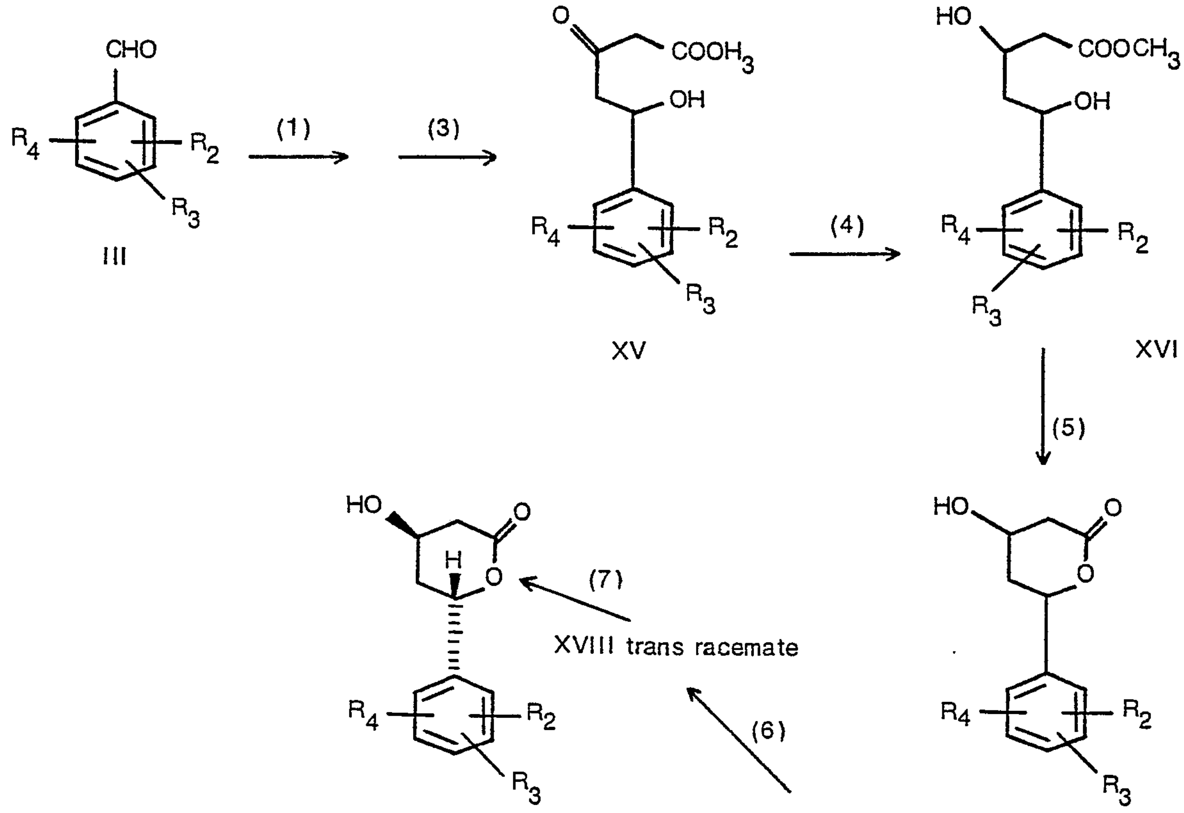


III

XV

XVI

XVII

XVIII trans racemate

XIX 4 *R* trans enantiomorph

B. Methylene Bridge

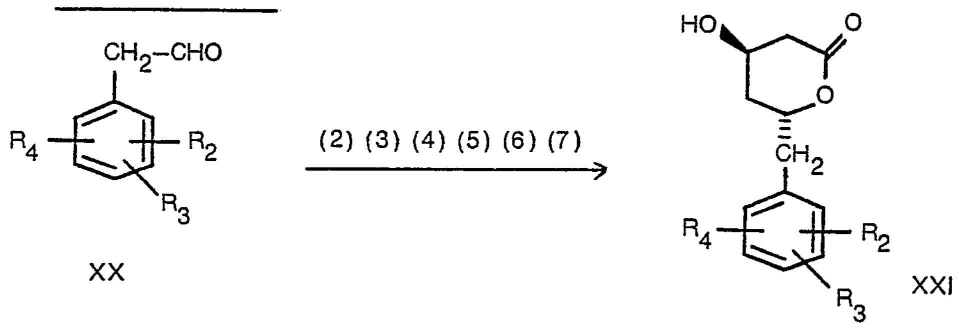


XX

XXI

C. Ethylene Bridge

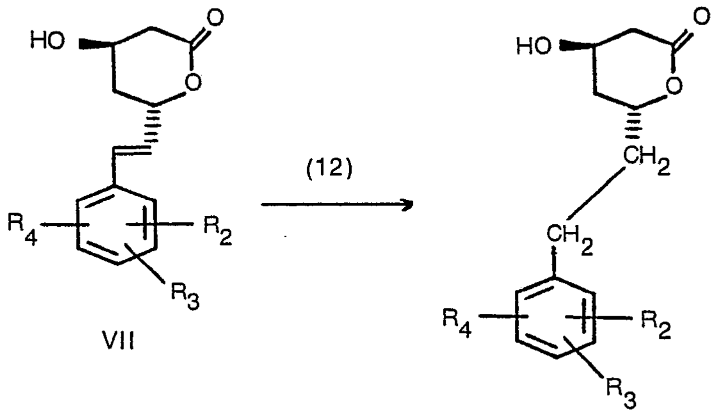


VII

XXII

D. Propylene Bridge

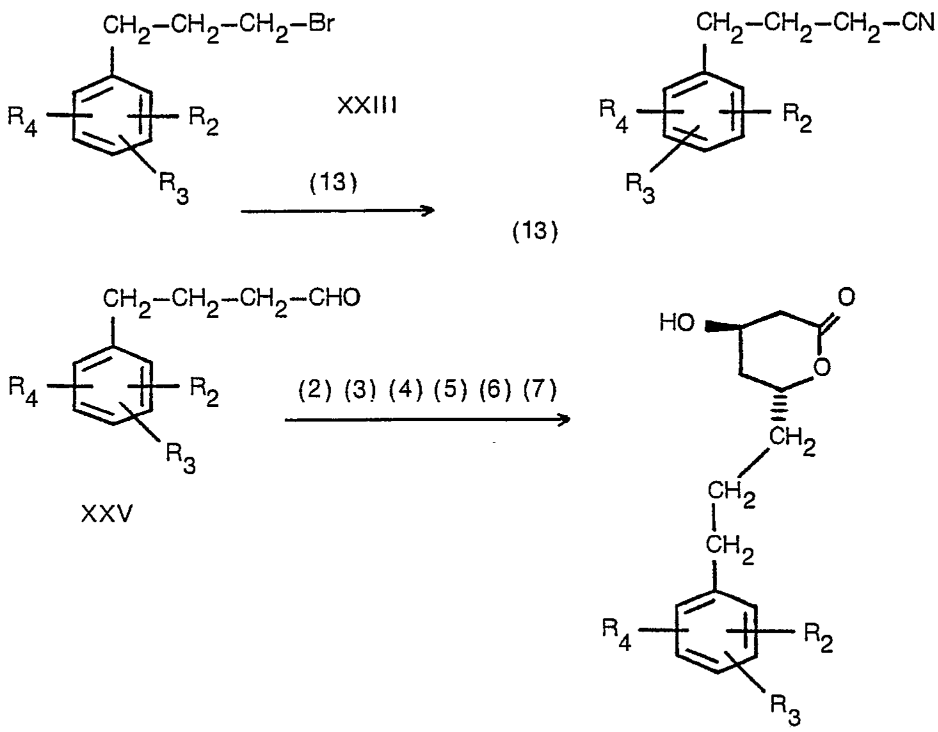


XXIII

XXIV

XXV

XXVI

Definitions: $R_2$, $R_3$, $R_4$, as defined in specifications.

## FLOW SHEET IV

Synthesis of Substituted 4-Hydroxy-4-Methyl-3,4,5,6-Tetrahydro-2H-Pyran-2-Ones

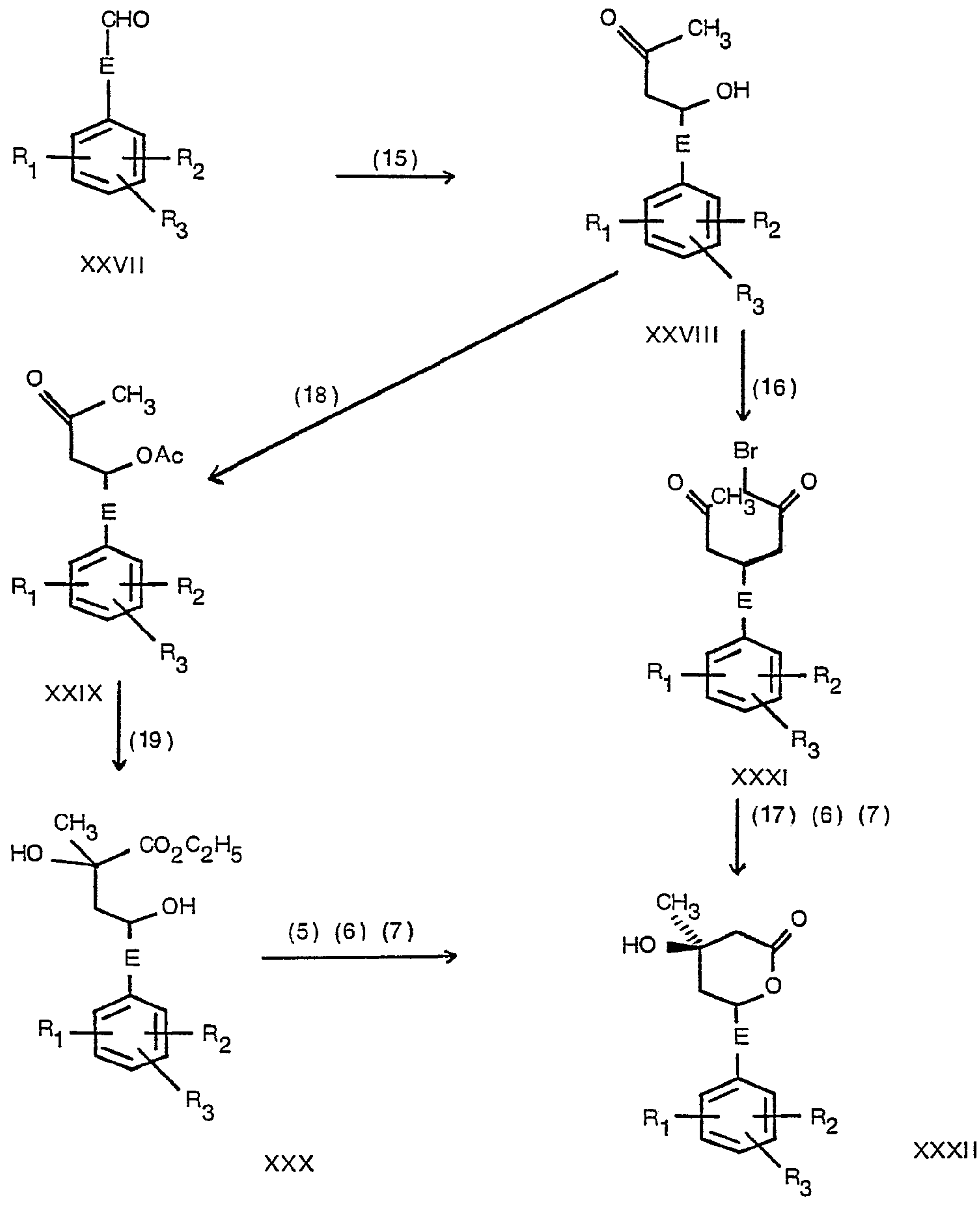


XXVII XXVIII XXIX XXXI XXX XXXII

DEFINITIONS: E, $R_1$, $R_2$ and $R_3$ are defined in the specifications given for Formula I.

REACTIONS IN FLOW SHEETS I—IV

1. When $R_1$, $R_2$ or $R_3$ is HO— or bears a hydroxyl substituent, the HO— group is etherified using a reagent $R_4X$ in a suitable solvent such as DMF and the like in the presence of a suitable base, preferably an alkali metal carbonate such as $K_2CO_3$, to give the corresponding ether $R_4O$— which can be carried through the remainder of the synthesis. If it is desired to remove $R_4$ at a later synthesis step, $R_4$ is chosen as an easily removable group such as $CH_3OCH_2CH_2OCH_2$— (the MEM protecting group). The MEM group is removed readily by treatment with a Lewis acid catalyst such as $ZnBr_2$ in a suitable solvent such as $CH_2Cl_2$ and the like. When the starting material is devoid of a hydroxyl group, step (1) is omitted.

2. *Aldol Reaction.* This can be run in several ways:

a) *The classical Aldol synthesis* in which acetaldehyde is condensed with the starting benzaldehyde, the resulting β-hydroxyaldehyde is acetylated with acetic anhydride and acetic acid is eliminated thermally to give the corresponding cinnamaldehyde.

b) *The directed Aldol condensation* in which the anion of an appropriately N-substituted ethylidenylimine, such as ethylidenecyclohexylimine and the like is condensed with the starting benzaldehyde at or below room temperature in an aprotic solvent, such as THF and the like, to afford a β-hydroxy-β-phenylpropylidenylimine which, upon concomitant dehydration and imine hydrolysis in an acidic medium, such as dilute aqueous HCl, provides the corresponding cinnamaldehyde.

c) The use of a *nucleophilic acetaldehyde equivalent* in which *cis*-2-ethoxyvinyllithium, generated from *cis*-1-ethoxy-2-tri-*n*-butylstannylethylene, is condensed with starting benzaldehyde to give an allylic alcohol which is subsequently rearranged, under suitable acidic conditions, to the corresponding cinnamaldehyde.

3. *Dianion Step.* Reaction with the dianion of acetoacetic ester in a suitable aprotic solvent such as THF, dioxane and the like.

4. *Reduction* with $NaBH_4$ in a suitable solvent such as methanol, ethanol and the like at or below room temperature.

5. *Lactonization.* Saponification by base (e.g. NaOH) in aqueous alcohol followed by acidification and cyclodehydration by heating in toluene.

NOTE: Steps 3, 4 and 5 are usually carried out sequentially without purification of compounds V and VI.

6. *Separation of the cis and trans* racemic mixtures by chromatography on silica gel or crystallization.

7. Resolution of the *trans* racemate into its enantiomers by treating the (±)-*trans* lactone with either *d*-(+) or *l*-(—)-α-methylbenzylamine to give the diastereomeric dihydroxy amides which are separated by chromatography or crystallization. Hydrolysis of each pure diastereomeric amide under basic conditions, such as ethanolic NaOH and the like, affords the corresponding enantiomerically pure dihydroxy acid which, upon lactonization, e.g., in refluxing toluene, provides the pure (+)-*trans* or (—)-*trans* enantiomer. Stereochemistry depends on the absolute stereochemistry of the diastereomeric amide from which it is derived.

8. Saponification with $M^{+-}$ OH where $M^+$ is an alkali metal cation.

9. Careful acidification.

10. Mild hydrolysis.

11. Nucleophilic opening of the lactone ring with an alcohol, $R_5OH$, in the presence either of a basic catalyst particularly the corresponding alkoxide, $R_5O^-$, or an acidic catalyst such as an acidic ion exchange resin, e.g. *Amberlite 120.*

12. Hydrogenation in the presence of a suitable catalyst such as Rhodium or Palladium on carbon.

13. Reaction with NaCN in a suitable solvent such as aqueous ethanol and the like.

14. Reduction with DIBAH in a aprotic solvent such as toluene, ether, THF and the like followed by work up with an aqueous acid such as 5% $H_2SO_4$.

15. Aldol condensation with 1-(tri-n-butyl stannyl) propan-2-one.

16. Acylation with 2-bromoacetyl bromide.

17. Intramolecular Reformatsky reaction carried out for example, in the presence of activated zinc dust, cuprous bromide and diethylaluminum chloride.

18. Acylation with acetyl chloride.

19. Intermolecular Reformatsky reaction carried out with ethyl 2-bromacetate, for example, in the presence of the reagents indicated in step (17) above.

The preparation of a typical inhibitor of this type can be described as follows:

Step A. Preparation of 2,4-Dichloro-6-phenylmethoxybenzaldehyde

Potassium carbonate (9.4 g, 67.8 mmole) was added to a stirred solution of 4,6-dichlorosalicylaldehyde (10.8 g, 56.5 mmole) in dimethylformamide (80 ml). The resulting mixture was stirred at 60° for 30 minutes and treated with benzyl bromide (10.6 g, 62.1 mmole). This mixture was stirred one hour at 60°C and then poured into ice water (1000 ml) to give the above named compound (15.9 g, 100%) which melted at 98—100°C after recrystallization from hexane. pmr ($CDCl_3$) α 5.10 (2H, s), 7.33 (5H, s), 10.40 (1H, s).

Analysis Calc. for $C_{14}H_{10}Cl_2O_2$
Calc.: C, 59.81; H, 3.58
Found: C, 59.98; H, 3.58.

Step B. Preparation of (E)-2,4-Dichloro-6-phenylmethoxycinnamaldehyde

A stirred suspension of 2,4-dichloro-6-phenylmethoxybenzaldehyde (15.5 g, 55.1 mmole) in acetaldehyde (30 ml) was cooled to 5°C and treated with 25% methanolic potassium hydroxide (1.4

ml, 6.24 mmole) at such a rate that the internal temperature was maintained at 25—30°C. The resulting solution was stirred for 30 minutes in the ice bath, treated with acetic anhydride (30 ml) and then heated at 100°C for 30 minutes. After cooling to 30°C the solution was treated with water (84 ml) and 12N hydrochloric acid (7 ml). The resulting mixture was refluxed for 30 minutes and then cooled in an ice bath to give a gummy solid which was recrystallized from cyclohexane to give the above named compound (5.6 g, 33%), mp 109—112°C. pmr ($CDCl_3$) $\alpha$ 5.10 (2H, s), 7.33 (5H, s), 9.68 (1H, d).

Analysis Calc. for $C_{16}H_{12}Cl_2O_2$
Calc.: C, 62.56; H, 3.94
Found: C, 62.66; H, 3.98.

Alternate Step B. Preparation of (E)-2,4-Dichloro-6-phenylmethoxycinnamaldehyde

A 1.6M solution (18.8 ml, 30 mmole) of n-butyllithium in hexane was added cautiously to a stirred solution of freshly distilled diisopropylamine (3.0 g, 30 mmole) in anhydrous tetrahydrofuran (200 ml) maintained at 0°C under a nitrogen atmosphere. The resulting solution was stirred at 0°C for 15 minutes and then treated with ethylidenecyclohexylamine (3.75 g, 30 mmole). The solution was stirred 15 minutes at 0°C, cooled to —78°C and treated with a solution of 2,4-dichloro-6-phenylmethoxybenzaldehyde (8.4 g, 30 mmole) in an anhydrous tetrahydrofuran (50 ml). The resulting red solution was stirred at —78°C for 15 minutes and at 25°C for 60 minutes. The reaction solution was treated with water (200 ml) and extracted with ether (3 x 200 ml). The organic extracts were combined, washed with brine (3 x 100 ml), dried over magnesium sulfate, and filtered. The filtrate was evaporated *in vacuo*, leaving the desired intermediate imine as a brown viscous oil (12.5 g) pmr ($DCCl_3$) $\alpha$ 5.10 (2H, s), 5.50 (1H, t), 7.37 (5H, s), 7.70 (1H, s).

A solution of the oily imine (12.5 g) in tetrahydrofuran (110 ml) was treated with a solution of oxalic acid dihydrate (11 g, 87.2 mmole) in water (22 ml). The resulting solution was refluxed for 30 minutes, cooled to 25°C and poured into water (500 ml). The resulting mixture was extracted with ether (3 x 200 ml). The organic extracts were combined, washed with brine (3 x 50 ml), dried over magnesium sulfate and filtered. The filtrate was evaporated *in vacuo*, leaving the above named compound as a tan solid. The title compound was purified by recrystallization from cyclohexane to give yellow needles (4.7 g, 51%) melting at 109—111°C, pmr ($CDCL_3$) a 5.11 (2H, s), 7.33 (5H, s), 9.68 (1H, d).

Step C. Preparation of (E)-Methyl 7-(2,4-dichloro-6-phenylmethoxyphenyl)-5-hydroxy-3-oxo-6-heptenoate

Methyl acetoacetate (9.56 g, 82.3 mmole) was added dropwise to a stirred suspension of sodium hydride (50% oil suspension) (3.95 g, 82.3 mmole) in anhydrous tetrahydrofuran at 0°C under a nitrogen atmosphere. The resulting solution was stirred 15 minutes at 0°C and then treated with a 1.6M solution (51.5 ml, 82.3 mmole) of n-butyllithium in hexane over 5 minutes. The resulting yellow solution was stirred 15 minutes at 0°C and then treated with a solution of (E)-2,4-dichloro-6-phenylmethoxycinnamaldehyde (25.3 g, 82.3 mmole) in anhydrous tetrahydrofuran (150 ml). The resulting orange solution was stirred 15 minutes at 0°C and then quenched by a dropwise addition of 12N hydrochloric acid (ca. 20 ml). The reaction mixture was diluted with water (100 ml) and extracted with ether (3 x 300 ml). The organic extracts were combined, washed with brine (3 x 100 ml), dried over magnesium sulfate and filtered. The filtrate was evaporated *in vacuo*, leaving the above named compound as a yellow oil (34.8 g, 100%), pmr ($CDCl_3$) $\alpha$ 2.75 (2H, d), 3.45 (2H, s), 3.72 (3H, s), 4.71 (1H, m), 5.50 (2H, s), 7.37 (5H, s).

Step D. Preparation of (E)-Methyl 7-(2,4-Dichloro-6-phenylmethoxyphenyl)-3,5-dihydroxy-6-heptenoate

Sodium tetrahydridoborate (1.55 g, 41.1 mmole) was added with stirring to a cooled solution (5°C) of (E)-methyl 7-(2,4-dichloro-6-phenylmethoxy-phenyl)-5-hydroxy-3-oxo-6-heptenoate (34.8 g, 82.3 mmole) in ethanol (200 ml) at a rate sufficient to maintain the internal temperature at 20 ± 5°C. The resulting solution was stirred in the ice bath for 15 minutes and then acidified with 6N hydrochloric acid. The resulting mixture was diluted with water (500 ml) and extracted with ether (3 x 250 ml). The organic extracts were combined, washed with brine (4 x 100 ml) dried over magnesium sulfate and filtered. The filtrate was evaporated *in vacuo*, leaving the above named compound as a yellow oil (34.8 g, 99.5%). pmr ($CDCl_3$) $\alpha$ 2.45 (2H, d), 3.65 (3H, s), 4.18 (1H, m), 4.45 (1H, m), 4.98 (2H, s), 7.28 (5H, s).

Step E. Preparation of (E)-7-(2,4-Dichlorophenyl-methoxyphenyl)-3,5-dihydroxy-6-heptenoic acid

A solution of (E)-methyl 7-(2,4-dichloro-6-phenylmethoxyphenyl)-3,5-dihydroxy-6-heptenoate (34.8 g, 81.8 mmole), 1N sodium hydroxide (82 ml, 82 mmole) and ethanol (200 ml) was stirred at 25°C for 15 minutes. The reaction solution was acidified with 6N hydrochloric acid, diluted with water (400 ml) and extracted with ether (3 x 200 ml). The combined organic extracts were washed with

brine (3 × 100 ml), dried over magnesium sulfate and filtered. The filtrate was evaporated *in vacuo,* leaving the above named compound as an orange oil (33.3 g, 99%). pmr ($CDCl_3$) $\alpha$ 2.47 (2H, d), 4.30 (2H, bm), 4.98 (2H, s), 7.30 (5H, s).

Step F. Preparation of (E)-6-[2-(2,4-Dichloro-6-phenylmethoxyphenyl)ethenyl]-3,4,5,6-tetrahydro-4-hydroxy-2H-pyran-2-one

A solution of (E)-7-(2,4-dichlorophenyl-methoxyphenyl)-3,5-dihydroxy-6-heptenoic acid (33.3 g, 81.3 mmole) in toluene (300 ml) was heated at reflux in a Dean-Stark apparatus. After 2 hours the Dean-Stark apparatus was replaced with a soxhlet containing 3Å molecular sieves (100 g). The solution was refluxed for an additional 4 hours and then the toluene was removed *in vacuo* leaving a yellow oil (31.7 g) which is a mixture of cis and trans isomers of the title compound. The oil was chromatographed on a silica gel column (900 g). Elution with methylene chloride-acetone (9:1, V:V; 4000 ml) provided a forerun which was discarded. Continued elution with the same eluant (500 ml) gave the trans isomer of the above named compound as a pale yellow solid (5.8 g).

Further elution of the column with the same eluant (3250 ml) gave a tan solid (8.8 g), which is a mixture of the cis and trans isomers. This cis/trans mixture was chromatographed using a Waters Prep LC500. The separation was accomplished by using two prep PAK—500/silica cartridges in series and eluting with methylene chloride-acetone (9:1, V:V). By using the shave-recycle technique the cis (4.7 g) and the trans (3.3 g) isomers were separated. The fractions of the trans isomer, collected from the two chromatographies, were combined and recrystallized from n-butyl chloride to give the trans isomer (7.3 g, 23%). pmr ($CDCl_3$) $\alpha$ 5.07 (2H, s), 5.30 (1H, m), 7.42 (5H, s).

Analysis Calc. for $C_{20}H_{18}Cl_2O_4$
Calc.: C, 61.08; H, 4.61
Found: C, 61.12; H, 4.60.

The cis isomer melted at 130—131.5°C after recrystallization from n-butyl chloride (4.3 g, 13%). pmr ($CDCl_3$) $\alpha$ 4.30 (1H, m), 4.83 (1H, m), 5.12 (2H, s), 7.47 (5H, s).

Analysis Calc. for $C_{20}H_{18}Cl_2O_4$
Calc.: C, 61.08; H, 4.61
Found: C, 61.55; H, 4.63.

Step G. Preparation and Separation of Diastereomeric Amides

A solution of (±)trans-(E)-6-[2-(2,4-dichlorophenyl)ethenyl]-3,4,5,6-tetrahydro-4-hydroxy-2H-pyran-2-one (2.87 g, 10 mmole) in d-(+)$\alpha$-methylbenzylamine (15 ml) was stirred for 18 hours at 25°C and then poured into water (100 ml). This aqueous mixture was acidified with 6N hydrochloric acid and then extracted with ether (3 × 100 ml). The ether extracts were combined, washed with brine (4 × 75 ml), dried over magnesium sulfate and filtered. Evaporation of the filtrate *in vacuo* gave the intermediate diastereomeric amides as a tan viscous oil (4.1 g).

The tan viscous oil (3.1 g, 7.6 mmole) was chromatographed on a silica gel column (200 g). Elution with acetone-methylene chloride (1:4, V:V, 1200 ml) gave a forerun which was discarded. Continued elution with the same eluant (1000 ml) gave the diastereomeric amides as a viscous oil (3.0 g).

The diastereomeric amides were separated by chromatography on a Waters Prep LC500. The separation was accomplished by using two prep PAK—500 silica cartridges in series and eluting with acetonemethylene chloride (1:4, V:V). By using the shave-recycle technique isomer A (1.36 g) and isomer B (1.20 g) were obtained.

Recrystallization of isomer A from n-butyl chloride gave colorless clusters (1.0 g) which melted at 106—108°C, pmr ($CDCl_3$) $\alpha$ 1.47 (3H, d), 2.33 (2H, d), 4.30 (1H, m), 5.17 (1H, q), 7.33 (8H, m).

Analysis Calc. for $C_{21}H_{23}Cl_2NO_3$
Calc.: C, 61.77; H, 5.68; N, 3.43
Found: C, 61.78; H, 5.78; N, 3.50.

Recrystallization of isomer B from n-butyl chloride-petroleum ether gave a pale yellow solid which melted at 55—60°C, pmr ($CDCl_3$) $\alpha$ 1.47 (3H, d), 2.33 (2H, d), 4.30 (1H, m), 5.17 (1H, q), 7.33 (8H, m).

Analysis Calc. for $C_{21}H_{23}Cl_2NO_3$
Calc.: C, 61.77; H, 5.68; N, 3.43
Found: C, 61.41; H, 5.87; N, 3.30.

Step H. Preparation of (+)trans-(E)-6-[2-(2,4-Dichlorophenyl)ethenyl]-3,4,5,6-tetrahydro-4-hydroxy-2H-pyran-2-one

Isomer A (0.74 g, 1.8 mmole) of Step G was dissolved in 95% ethanol (25 ml) containing 1N sodium hydroxide (3.6 ml, 3.6 mmole) and the solution was refluxed for 54 hours. The ethanol was removed *in vacuo* and the residue was suspended in water (100 ml) and acidified with 6N hydrochloric acid. This aqueous mixture was extracted with ether (3 x 75 ml). The ether extracts were combined, washed with brine (2 x 50 ml), dried over magnesium sulfate, and filtered. The filtrate was evaporated *in vacuo* leaving the intermediate acid as a yellow oil (0.54 g).

A solution of the yellow oil in toluene (150 ml) was refluxed through a soxhlet containing molecular sieves (3Å) for 5 hours. The solution was evaporated *in vacuo* leaving the (+) trans isomer as a yellow solid. The title compound was purified by recrystallization from ether and then n-butyl chloride to give white needles (0.11 g, 20%) melting at 114—115°C, pmr ($CDCl_3$) $\alpha$ 2.03 (2H, m), 2.73 (2H, m), 4.46 (1H, m), 5.41 (1H, m), 6.19 (1H, dd), 7.01 (1H, d), 7.14—7.50 (3H, m).

Analysis Calc. for $C_{13}H_{12}Cl_2O_3$
Calc.: C, 54.37; H, 4.21
Found: C, 54.51; H, 4.32.

$[\alpha]_D^{25} = +5.9$ (C, 0.425, chloroform)

Step I. Preparation of (—)trans-(E)-6-[2-(2,4-Dichlorophenyl)ethenyl]-3,4,5,6-tetrahydro-4-hydroxy-2H-pyran-2-one

Isomer B (1.1 g, 2.7 mmole) of Step G was dissolved in 95% ethanol (25 ml) containing 1N sodium hydroxide (5.4 ml, 5.4 mmole) and the solution was refluxed for 18 hours. The ethanol was removed *in vacuo* and the residue was suspended in water (100 ml) and acidified with 6N hydrochloric acid. This aqueous mixture was extracted with ether (2 x 100 ml). The ether extracts were combined, washed with brine (3 x 50 ml), dried over magnesium sulfate and filtered. The filtrate was evaporated *in vacuo* leaving the intermediate acid as a yellow oil (0.85 g).

A solution of the yellow oil in toluene (150 ml) was refluxed through a soxhlet containing molecular sieves (3Å) for 5 hours. The solution was evaporated *in vacuo* leaving the (—) trans isomer as a yellow solid. The compound was recrystallized twice from n-butyl chloride to give white needles (0.34 g, 44%) melting at 114—115°C, pmr ($CDCl_3$) $\alpha$ 2.03 (2H, m), 2.73 (2H, m), 4.46 (1H, m), 5.41 (1H, m), 6.19 (1H, dd), 7.01 (1H, d), 7.14—7.50 (3H, m).

Analysis Calc. for $C_{13}H_{12}Cl_2O_3$
Calc.: C, 54.37; H, 4.21
Found: C, 54.31; H, 4.26.

$[\alpha]_D^{25} = -6.6$ (C, 0.555, chloroform)

*B. The Bile Acid Sequestrants*

The basic non-toxic anion exchange resins, which are the bile acid sequestrants used in this invention, may be water-soluble or water-insoluble or may swell in the presence of water. It is preferred to use these resins in salt form, for example, as a salt with a non-toxic inorganic acid such as hydrochloric acid, phosphorous acid or phosphoric acid. The resinous materials are used in fine particulate form and are preferably sieved so as to exclude oversize particles. The usable basic non-toxic anion exchange resins are:

1) styrene-divinyl-benzene copolymers which contain quaternary ammonium functional groups, the main constituent of which is polystyrene (Cholestyramine).

A particularly preferred type thereof is that in which basic groups have been introduced into a copolymer of styrene with a small proportion of divinylbenzene. The copolymer is subject to chlormethylation and the product then treated with a tertiary amine, preferably a trialkylamine containing not more than 10 carbon atoms, so as to introduce basic quaternary ammonium groups. The styrene-divinylbenzene copolymer preferably contains not more than 5 percent, preferably 1—4 percent of divinylbenzene. The extent of chlormethylation and hence the proportion of quaternary groups can be varied over quite wide limits. Such products have a high molecular weight and an equivalent weight based upon the ammonium groups, of less than 500, usually of about 175—200.

The most preferred synthetic, strongly basic anion exchange resin of this type is designated cholestyramine and contains quaternary ammonium functional groups which are attached to a styrene-divinyl-benzene copolymer. The main constituent is polystyrene trimethylbenzylammonium with $Cl^-$ anion, but it also contains divinylbenzene (2%) and water (0—75%). Cross-linkage %: 1—10. Particle size: 50—100 mesh having the typified structure of main polymeric groups:

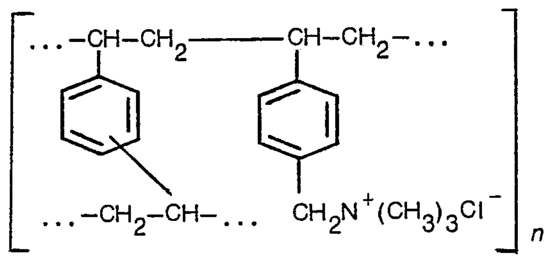

Compositions and methods for binding bile acids with cholestyramine are described in U.S. Patent No. 3,383,821 and 3,308,020.

2) Water-insoluble high molecular weight copolymers obtained from polyalkylene polyamines and epichlorohydrin, glycerol-1,3-dichlorohydrin, an aliphatic bisepoxy compound or an alpha-omega-alkylene glycol (*Colestipol*).

Examples for aliphatic bisepoxy compounds are 1,2: 3,4-bis-epoxybutane and bis-epoxypropyl ether. A preferred type of polyalkylene polyamine is a polyethylene polyamine and amongst the latter, diethylenetriamine is the preferred material. Such compounds contain at least as many secondary amino groups as primary amino groups in the molecule. The preferred polymer of this type designated colestipol, is a copolymer of diethylenetriamine and 1-chloro-2,3-epoxypropane and may be regarded as a cross-linked polymer of these two substances which is brought to a pH of approximately 4 with hydrochloric acid, then dialyzed and the dialyzed product dried. In the reaction products a proportion of the amino groups are quaternized and form chloride salts.

3) *Esters of crosslinked polysaccharides*

A third type of resin consists of the omega-dialkylaminoalkyl, the omega-di(hydroxyalkyl)aminoalkyl and the omega-morpholino alkyl ethers of cross-linked polysaccharides, for example, of dextran, starch and sucrose cross-linked with epichlorohydrin together with the non-toxic salts of such ethers with acids. The dextrans are anhydroglucose polymers produced by the action of *Leuconostoc mesenteroides* upon aqueous solutions of sucrose. The copolymer of dextran and epichlorohydrin has from 10 to 35% of its weight of hydroxyl groups, based upon the dry substance, from 6 to 50 percent of said hydroxyl groups are replaced by groups of the formula RYO— in which Y is an alkylene group having at least one but less than three carbon atoms and R is a dialkylamino group having at least two but less than five carbon atoms, said copolymer having an ion exchange capacity of 2 to 6 milliequivalents per gram of dry compound. Preferred compounds of this type are those containing 2-diethylaminoethyl, 2-dimethylaminoethyl, 2-diethylaminomethyl, di(hydroxyethyl)-aminoethyl, di(hydroxyethyl)aminoethyl, $\beta$-morpholinoethyl and $\alpha$-morpholinomethyl groups. Such compounds are, for example, described in U.S. Patent No. 3,277,025, together with their methods of production. The preferred salts of these compounds are the hydrochlorides.

These compounds are insoluble in water but swell therein and have a water regain of from 1 to 50 grams per gram of dry compound.

A preferred polymer of this type is dextran cross-linked with epichlorohydrin which is subjected to after treatment with diethylaminoethyl halide in the presence of an aqueous alkali such as caustic soda the product may be characterized as poly[2-(diethylamino)ethyl]polyglycerylene dextran.

4) Quaternary ammonium anion exchange resins having a linear backbone which is free from both branching and cross-linking, comprising quaternized nitrogen atoms linked to each other through trimethylene groups (*Ionenes*).

By the term "linear backbone" is meant that the polymer has only acyclic groups, i.e. trimethylene, linking the nitrogen atoms in a single continuous chain; the polymer is free from "branching" when it has no repeating monomer units extending from the polymer backbone; and it is free from "cross-linking" when there is no joining of two linear backbones. The polymers are obtained by the polymerization of dihydro-oxazine, reductive alkylation of the resulting polymer, followed by quaternization.

Such compounds are for example described in U.S. Patent No. 4,098,726, together with their method of production.

A preferred such polymer is designated poly[methyl-(3-trimethylammoniopropyl)imino-trimethylene dihalide] and has the structural formula:

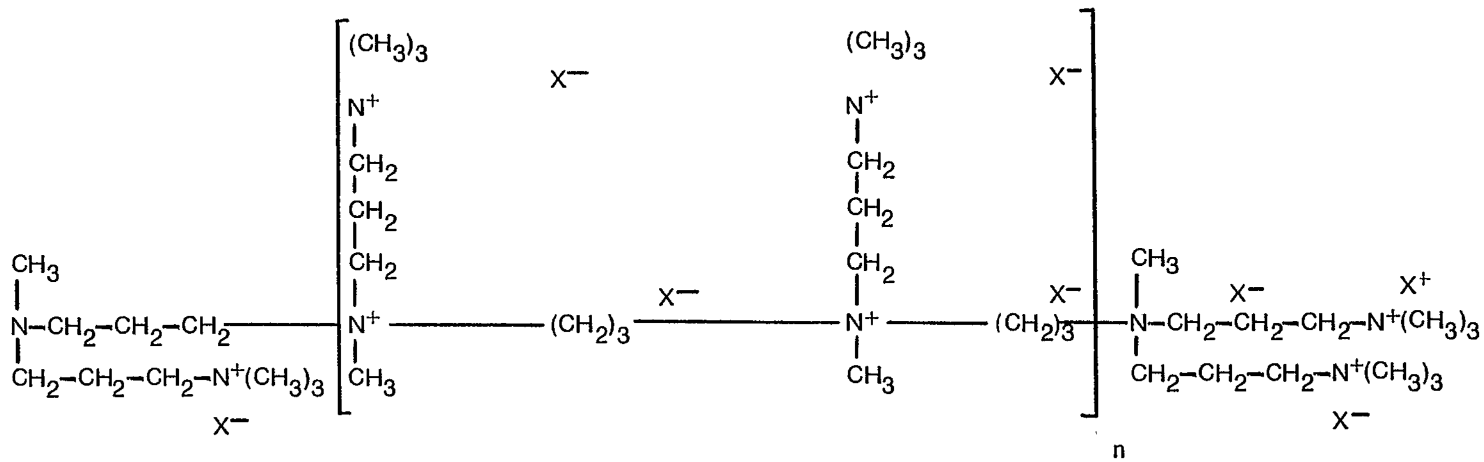

wherein $X^-$ is a halide anion.

This type of bile acid sequestrant includes water soluble quaternary ammonium linear polymers of monomers having the structural formulae:

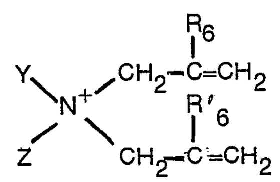

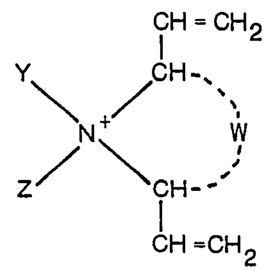

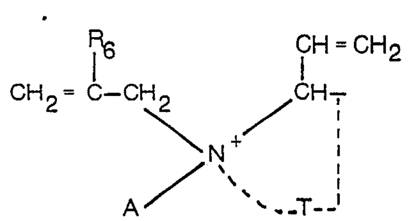

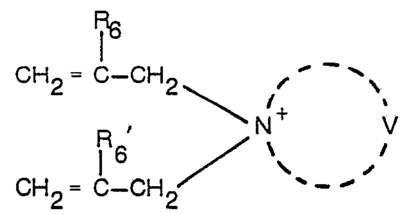

The nomenclature here employed, and in the claims, for the preferred polymer, which designates the quaternary nitrogen in the polymeric chain as "iminio", is in accord with the nomenclature now in use by Chemical Abstracts. The nomenclature sometimes used for this polymer elsewhere in the specification, including the illustrative experimental examples, which designates the quaternary nitrogen in the polymeric chain as "imino", is in accord with that approved by IUPAC Macromolecular Nomenclature Commission, as reported in Macromolecules, Vol. 6, No. 2, page 149 (1973).

It will be appreciated that, with each such cationic ammonium group, there is associated a chloride anion.

In the above formulae, the symbols Y and Z independently represent an alkyl, hydroxyalkyl, or phenyl radical which may contain as substituents such groupings as amido, carboloweralkoxy, loweralkoxy, mono- and dicyclic aryloxy, cyano, thioloweralkoxy, thiophenoxy, or lower alkoyl (forming a ketonic group) radicals, 5- and 6-membered cycloalkyl groupings, and, on the alkyl groupings only, a nitro group, and on the phenyl radical only, a halogen atom (chlorine, bromine, fluorine, and iodine).

The symbols $R_6$ and $R_6'$ independently represent a hydrogen, chloro, bromo, or lower alkyl or phenyl radical, having substituents as stated under the definition for Y and Z above.

The symbol W stands for a divalent radical of the formula:

$$—CH_2—(O)_n—(CH_2)_m—$$

The symbol T stands for a divalent radical of the formula:

$$—(CH_2)_p—(O)_n—CH_2—$$

The symbol V stands for a divalent radical of a formula:

$$—(CH_2)_p—(O)_n—(CH_2)_2—$$

In these last-mentioned formulae, the small letter n represents one of the numbers 0 to 1; the small letter m represents one of the numbers 1 and 2; and the small letter p represents one of the numbers 2 and 3.

The preferred water-soluble polymers of this type are the linear homopolymers of diallyldimethylammonium chloride hereinafter also referred to as Cat-Floc[R].

Such polymers are described, together with the method of preparation, in U.S. Patent 3,288,770.

5) *Water Soluble Anion Exchange Resin*

Also included in the present invention are further compositions containing the cholesterol synthesis inhibitors and water-soluble polymers which successfully bind bile acids. The water-soluble polymers have certain very definite characteristics which serve to delineate those which are operative. They must have a molecular weight greater than 1500, although polymers having a molecular weight above 3000 are preferred as cholesterol blood level reducing agents. Such materials are not absorbed in the alimentary tract. The polymers must have a plurality of ionizable amines (i.e., either quaternary amino groups, which are already ions, or amino groups sufficiently basic to form acid addition salts) in

contrast to pseudoamino groups such as amide nitrogen. Further, these amino groups must be sufficiently numerous in the polymer chain to make the equivalent weight based on titration of the amino groups less than 500. Finally, the polymer skeleton (i.e., the fundamental atom chain of the polymer) must be inert to degradation by digestive enzymes. Since the latter act principally by hydrolysis, this means that the polymer must be free of easily hydrolyzed amide, ester and the like, linkages. Above all, they must meet the in vitro test of binding at least 40% of glycocholic acid.

Various polymeric substances of this type are useful according to the present invention. One such polymer, variously known as "Acryloid CQ" and "Acrysol CQ", a linear acrylic type quaternary ammonium salt polymer having a molecular weight on the order of about 2,000,000, made by Rohm and Haas Company, Philadelphia, Pa., is quite useful in reducing cholesterol blood levels. This polymer, structurally a straight carbon skeleton with ester side chains, the esters being from quaternary ammonium substituted alcohols, is soluble in water, and the water solution has a viscosity of 2500 to 5000 centipoises in 5% aqueous solution at room temperature and available from the manufacturer in an aqueous solution containing about 12 to 14 percent by weight of polymer. It has an equivalent weight, based on the ammonium groups, of about 350 to 360.

Another polymer which is effective according to the present invention is "Acrysol CA", a soluble tertiary amine salt available from Rohm and Haas Company. Aside from the fact that this polymer is a tertiary amine salt rather than a quaternary ammonium salt, its properties in general are the same as those of Acrysol CQ. Its equivalent weight is of the order of 325.

A further water-soluble polymer of this type which is useful in cholesterol therapy according to this invention is polyethyleneimine, which has the structure

$$(-CH_2CH_2NHCH_2CH_2NH-)_n$$

and an average molecular weight of about 30,000. This polymer is available from the Borden Company, New York, N.Y. It has a very low equivalent weight, of the order of about 43, since each imine group is an active ionizable amine.

Still another water-soluble polymer useful in cholesterol blood level reduction according to the present invention is "Separan CR70", made by the Dow Chemical Co., Midland, Michigan. This material is a copolymer of acrylamide and vinylbenzyltrimethylammonium chloride in a weight ratio of about 30:70, having an equivalent weight of about 302 and an average molecular weight of above 100,000. Still further examples of the above water-soluble polymers are described in U.S. Patent No. 3,308,020.

C. *The Composition of this Invention*

The composition of this invention comprise (1) one or more cholesterol synthesis inhibitors acting at the HMG—CoA reductase level with an $ED_{50}$ less than $10^{-6}$ M and (2) one or more polymeric anion exchange bile acid sequestrants, each especially of the types described above. The two components can also be coadministered in the method of this invention.

The most preferred composition is that of compounds III and/or III' with cholestyramine. However, compositions of cholestyramine with compounds II and/or II' or IV and/or IV' are also important aspects of this invention.

The compositions in which colestipol are used with, preferably compound III and/or III', but also II and/or II' and IV and/or IV' are also important aspects of this invention.

As will be demonstrated hereinafter, the admixture of the cholesterol synthesis inhibiting compounds with the above non-toxic anion exchange resins has a synergistic effect in the reduction of cholesterol values in plasma. To obtain the synergistic advantage from the compositions it is preferred to use mixtures of compound (I) or (I') to resin in the proportion of from 1:1 to 1:1500 by weight.

Resins of the above kinds are known to be capable of sequestering bile acids in the intestine and are then excreted in the feces. However, there is a wide variation amongst patients. The resins are, in any event, only moderately effective because the liver compensates to some degree by synthesizing more cholesterol.

The compositions of this invention are administered orally in the form of capsules, tablets and the like. It is usually desirable to use the oral route. Doses may be varied, depending on the age, severity, body weight and other conditions of human patients but daily dosage for adults is within a range of from about 2 mg to 2000 mg (preferably 10 to 100 mg) given in three or four divided doses. Higher doses may be favorably applied as required.

With respect to the treatment of patients suffering from hypercholesteremia, it is recommended that the active ingredients of the composition of the present invention be co-administered as separate dosages or, preferably, as a unitary combination dosage. The dosage of the compounds of formula I or I' may be from .1 to 25 mg/kg/day. That of the more potent compounds of formula III or III' may be from 0.02 to 10 mg/kg/day. For compounds of formula IV, a dosage of 0.2 to 50 mg/kg/day is used. In terms of a 70 kg average weight human, the daily dosage ranges are 7 to 1750 mg of the compounds of Formula I or I', 1.4 to 700 mg of the compounds of formula III or III' and 14 to 3500 mg of the compounds of formula IV. The preferred usages are 1—2 mg/kg/day or 0.07—0.14 g/day for I or I', 0.2—0.5 mg/kg/day or 0.02—0.04 g/day for III or III' and 2—4 mg/kg/day or 0.14—0.3 g/day for IV.

The usage of the bile acid sequestrant resins, especially of cholestyramine, is 3.5 to 21 g/day, preferably 12—15 g/day. For infants and children the weight must be taken into account, with a preferred dosage of 0.3—1.1 g/kg/day.

It is contemplated within the present invention that any of the dose ranges of the cholesterol synthesis inhibitors recited above can be combined with any of the dose ranges recited above for bile acid sequestrants. The ratios of the ingredients by weight thus vary from 1:0.08 to 1:15,000, for example from 1:0.08 to 1:266 or from 1:1 to 1:15,000. The doses of the composition can be administered in one to three equal daily doses. Preferred dosage compositions of this invention for one daily dose thus are 12—15 g of sequestrant resin and 0.7 to 0.14 g of compactin 0.02—0.04 g of methyl-compactin or 0.14—0.3 g of the compounds of formula IV. Unit dosages of one half or one third this size are used for multiple daily doses.

Protocol for Evaluating Combination of Compound (II) with Cholestyramine in Dogs

Male beagles, ranging in weight from 7 to 14 kg. (average 11 kg.), were fed a semi-synthetic diet six weeks prior to the start of the experiment and serum cholesterol levels determined colorimetrically each week in order to obtain base-line values for each dog. At the start of the treatment, animals were divided into three treatment groups. Group A (4 dogs) received 25 mg./kg./day of compound (II) mixed in the diet. Group B (4 dogs) received 12 g./dog/day of cholestyramine mixed in the diet. Group C (5 dogs) was the untreated control. Treatment was for four weeks, and plasma cholesterol was determined two times per week. After four weeks a combination of compound (II) and cholestyramine was given to dogs in Groups A and B mixed in the diet at 25 mg./kg. and 12 g./dog/day respectively for an additional four weeks. Cholesterol levels were determined two times per week during this period.

The results are shown in Fig. 1. The lower curve, indicated by the solid line shows the results obtained with Group A receiving 25 mg./kg./day of compound (II). There is a rapid reduction in plasma cholesterol level plateauing by four weeks at 36% of control. With Group B receiving 12 g./kg./day of cholestyramine shown by the dashed line, there is a reduction of 35% in plasma cholesterol after four weeks. The control dogs, Group C, shown in the upper curve with the dash and dot line have the same cholesterol levels after four weeks as at the start. Combining compound (II) and cholestyramine in the diets of groups A and B at 25 mg./kg. and 12 g./dog/day respectively for an additional four weeks (indicated by the arrow in Fig. 1) results in further marked reductions. In Group A after four weeks on the combination there is a further reduction to 80% of the initial values.

The results shown graphically in Fig. 1 are also shown in tabular form in Table I. Thus as is summarized in Table I, treating dogs for four weeks with compound (II) at 25 mg./kg./dog mixed in the diet (Group A) results in a decrease of plasma cholesterol ranging from 34 to 40% (average of 35.6%). Treating dogs for four weeks with cholestyramine at 12 g./dog/day mixed in the diet (Group B) results in a decrease in plasma cholesterol ranging from 33 to 40% (average of 35.3%). From this data it is expected that upon treating Group A dogs with cholestyramine at 12 g./dog/day in combination with compound (II) there should be an additional 35.3% decrease in plasma cholesterol or an average of 53.4 mg./dl. after four weeks on the combination. It is found, however, that the plasma cholesterol level is reduced to an average of 27 mg./dl. or 67.2% (range 65—76%) with the combination as compared with compound (II) alone. Similarly treating Group B dogs with compound (II) at 25 mg./kg./day in combination with cholestyramine should result in a decrease of 35.6% in plasma cholesterol from the levels of the cholestyramine treatment alone to an average of the cholestyramine treatment alone to an average of 54.9 mg./dl. It is found instead that the average decrease with the combination is 58% (range 43—69%) resulting in an average plasma cholesterol of 35.8 mg./dl. Thus treating dogs with a combination of cholestyramine and compound II after they have been treated with either agent by itself results in a synergistic response to the combination.

TABLE I

PLASMA CHOLESTEROL
(mg./dl)

| | | | 4 Wks. Single Agent + 4 Wks. Combination | |
|---|---|---|---|---|
| Group | Start | 4 Weeks | Expected[a] | Found[b] |
| A | 120 | 76 (−36.7%) | 49.2 (−35.3%) | 24 (−68.4%) |
| | 101 | 70 (−30.7%) | 43.3 (−35.3%) | 17 (−75.7%) |
| | 155 | 92 (−40.4%) | 59.5 (−35.3%) | 31 (−66.3%) |
| | 140 | 92 (−34.3%) | 59.5 (−35.3%) | 32 (−65.2%) |
| average | 129 | 82.5 (−35.6%) | 52.9 (−35.3%) | 27.0 (−68.9%) |
| B | 150 | 102 (−34.0%) | 65.7 (−35.6%) | 32 (−68.6%) |
| | 145 | 87 (−40.0%) | 56.0 (−35.6%) | 43 (−50.5%) |
| | 111 | 74 (−33.3%) | 47.6 (−35.6%) | 42 (−43.2%) |
| | 118 | 78 (−33.9%) | 50.2 (−35.6%) | 26 (−66.7%) |
| average | 131 | 85.3 (−35.3%) | 54.9 (−35.6%) | 35.8 (−57.3%) |
| C | 109 | 102 (−6.4%) | | 113 (+10.8%) |
| | 140 | 134 (−4.3%) | | 129 (−3.7%) |
| | 108 | 103 (−4.6%) | | 108 (+4.9%) |
| | 118 | 115 (−2.5%) | | 115 (0) |
| | 145 | 132 (−8.9%) | | 137 (+3.8%) |
| average | 124 | 117 (−5.3%) | | 120.4 (+3.2%) |

[a] — Expected base on % decrease from treatment with single agent alone for 4 weeks.

[b] — % Decrease calculated from plasma cholesterol after 4 weeks treatment with single agent.

In a similar experiment, the effect of MSD 803 plus cholestyramine on dogs already treated with cholestyramine was found to be a reduction of plasma cholesterol by about 40%. Two beagle dogs were treated with 12 g/day of cholestyramine until the plasma cholesterol levels had decreased to the expected levels of 35% below pretreatment values, where continued treatment resulted in no further decrease. Administration of MSD 803 at a level of 4 mg/kg daily in combination with 12 g/day of cholestyramine resulted in an additional lowering of plasma cholesterol of 39.7% from the cholestyramine baseline. Thus in one dog the cholesterol level after treatment with cholestyramine was 87.8 mg/dl plasma. By 11 days of treatment with a combination of 12 g/day cholestyramine and 4 mg/kg/day of MSD 803 the plasma cholesterol levels had decreased to a new plateau which remained constant from day 11 to 32 at a level of 54, or 38.5% below the initial cholestyramine base. In a similar manner the plasma colesterol level of a second dog treated with cholestyramine was 94 mg/dl. This decreased to 55.6 mg/dl or 40.9% below the initial cholestyramine base after treatment with the combination of cholestyramine and 4 mg/kg MSD 803.

Example 1

A series of mixtures of powders of the following compositions, in which parts are by weight, are made.

| Ingredient | Parts Used | | |
|---|---|---|---|
| | A | B | C |
| Compound of Formula II | 0.11 | | |
| Compound of Formula III | | 0.03 | |
| 4 *R*-Trans-4-hydroxy-6-[2,4-dichloro-6-(4-fluorobenzyloxy)-phenyl]-3,4,5,6-tetra-hydropyran-2-one | | | 0.22 |
| Cholestyramine | 12 | 12 | 12 |
| Gum Arabic | 25 | 25 | 25 |
| Flavoring | 5 | 5 | 5 |
| Total Parts: | 42.11 | 42.03 | 42.22 |

The flavoring and gum arabic are first admixed and the other ingredients, in powder form, gradually admixed therewith. The mixture for oral use is given three times daily in doses of 14.6 grams each, mixed with water before taking.

Example 2

A series of mixtures similar to those in Example 1 except that the following are substituted in each mixture in equal quantity, for cholestyramine.

A. poly[2-(diethylamino)-ethyl]polyglycerylene dextran

B. Colestipol

C. poly[methyl-3-trimethylammoniopropyl]iminiotrimethylene dihalide

Example 3

The mixture of Examples 1 and 2 are each made into capsules containing 14 grams of the mixture. These are administered to a hypercholesteremic patient either 3 at a time daily or three a day spaced through the day.

Example 4

The mixtures of Examples 1 and 2 are each made into capsules containing 21 g. of the mixture. The capsules are administered to a hypercholesteremic patient, two a day, either in one dose or in separate doses.

Example 5

An aqueous dispersion of 42 grams of the mixture of Example 1B is administered daily to a patient suffering from hypercholesteremia.

**Claims for the Contracting States: BE CH DE FR GB IT LU NL SE**

1. A pharmaceutical composition for use in the treatment of hypercholesteremia, comprising

a) a cholesterol synthesis inhibitor acting at the 3-hydroxy-3-methylglutaryl-coenzyme A reductase level with a half level effective dose ($ED_{50}$) less than $10^{-6}$ which is selected from

$a_1$)

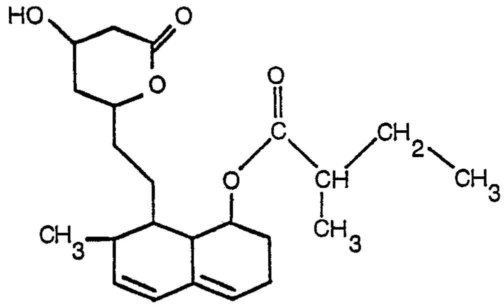

$a_2$)

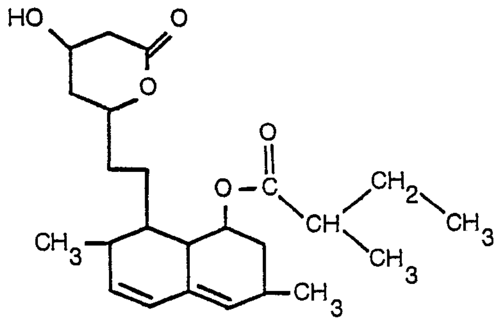

or

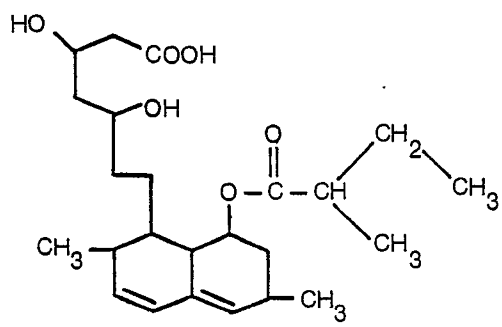

$a_3$) a (+) trans 6-E-4-hydroxy tetrahydropyran-2-one of the structure:

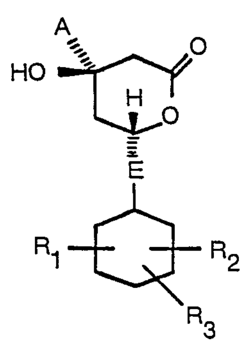

IV

wherein

A is H or methyl;

E is a direct bond, $—CH_2—$, $—CH_2—CH_2—$, $—CH_2—CH_2—CH_2—$ or $—CH{=}CH—$;

$R_1$, $R_2$, and $R_3$ are each selected from

H,

halogen,

$C_{1-4}$ alkyl,

$C_{1-4}$ haloalkyl,

phenyl,

phenyl substituted by

halogen,
$C_{1-4}$ alkoxy
$C_{2-8}$ alkanoyloxy
$C_{1-4}$ alkyl,
$OR_4$ in which $R_4$ is
H,
$C_{2-8}$ alkanoyl,
benzoyl,
phenyl,
phenyl $C_{1-3}$ alkyl,
cinnamyl,
$C_{1-4}$ haloalkyl,
alkyl, preferably $C_{1-9}$ alkyl,
$C_{3-6}$ cyclo alkyl-$C_{1-3}$-alkyl,
adamantyl, adamantyl-$C_{1-3}$-alkyl or
substituted phenyl $C_{1-3}$ alkyl in each of which the substituents are selected from
halogen,
$C_{1-4}$ alkoxy,
$C_{1-4}$ alkyl,
or
$C_{1-4}$ haloalkyl;

$a_4$) the corresponding hydroxy acids of the compounds of $a_1$) or $a_3$) in which the lactone ring is hydrolytically opened;

$a_5$) the pharmaceutically acceptable salts of said acids, and

$a_6$) the lower alkyl and phenyl dimethylamino or acetamino lower alkyl esters of said acids; and

b) a pharmaceutically acceptable nontoxic basic anion exchange resin capable of binding bile acids in a non-reabsorbable form in the gastro-intestinal tract which is selected from

$b_1$) styrene-divinyl-benzene copolymers which contain quaternary ammonium functional groups, the main constituent of which is polystyrene,

$b_2$) water-insoluble high molecular weight copolymers obtained from polyalkylene polyamines and epichlorohydrin, glycerol-1,3-dichlorohydrin, an aliphatic bisepoxy compound or an alpha, omega-alkylene glycol,

$b_3$) esters of crosslinked polysaccharides,

$b_4$) quaternary ammonium anion exchange resins having a linear backbone which is free from both branching and cross-linking, comprising quaternized nitrogen atoms linked to each other through trimethylene groups,

$b_5$) water soluble anion exchange resins with a molecular weight of greater than 1500 with a plurality of ionizable amines sufficiently numerous to make the equivalent weight based on titration of the amino groups less than 500, wherein the polymer skeleton must be inert to degradation by digestive enzymes, which meet the in vitro test of binding at least 40% of glycocholic acid,
said respective components being admixed in ratio between 1:0.08 and 1:15,000.

2. A composition of claim 1 in which said anionic exchange resin is a styrene-divinyl-benzene copolymer which contains quaternary ammonium functional groups, the main constituent of which is polystyrene trimethylbenzylammonium with $Cl^-$ anion.

3. A composition of claim 1 in which said anion exchange resin is a copolymer of diethylenetriamine and 1-chloro-2,3-epoxypropane.

4. The composition of claim 2 in which the cholesterol synthesis inhibitor is

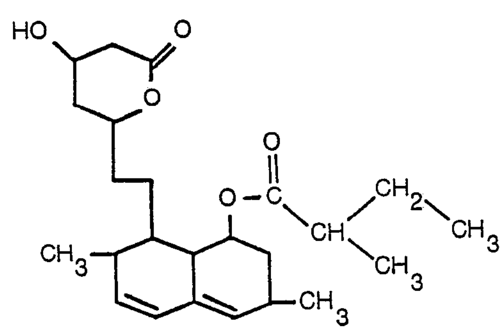

or

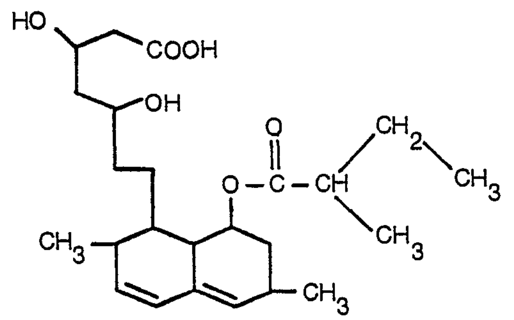

5. The composition of claim 2 in which the cholesterol synthesis inhibitor is

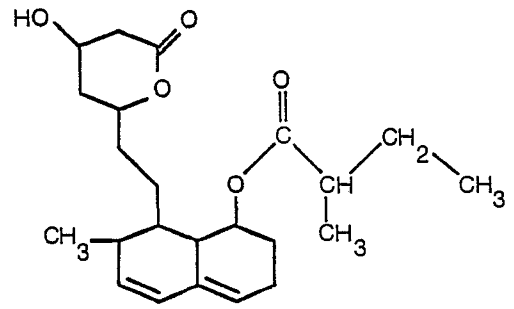

6. The composition of claim 1 in which said anion exchange resin is poly[methyl-(3-trimethylammoniopropyl)iminotrimethylene dihalide].

7. The composition of claim 6 in which the cholesterol synthesis inhibitor is

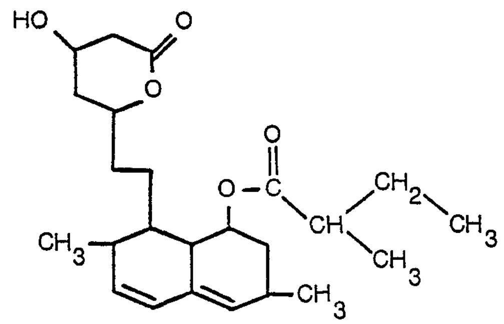

or

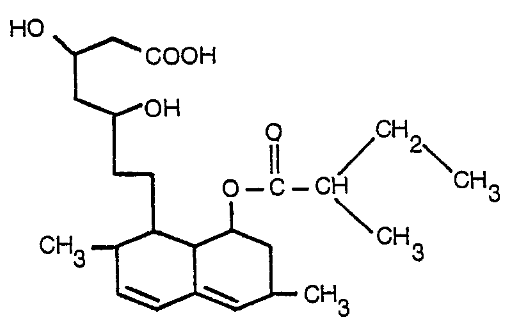

**Claims for the Contracting State: AT**

1. Process for preparing a pharmaceutical composition for use in the treatment of hypercholesteremia, by admixing

a) a cholesterol synthesis inhibitor acting at the 3-hydroxy-3-methylglutaryl-coenzyme A reductase level with a half level effective dose ($ED_{50}$) less than $10^{-6}$ which is selected from

$a_1$)

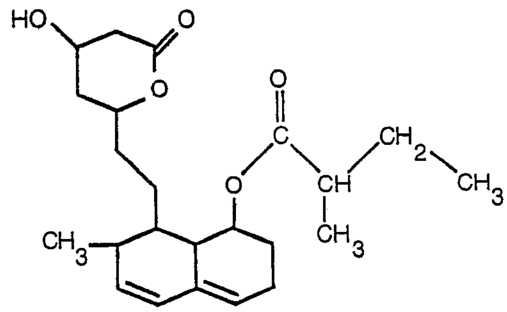

$a_2$)

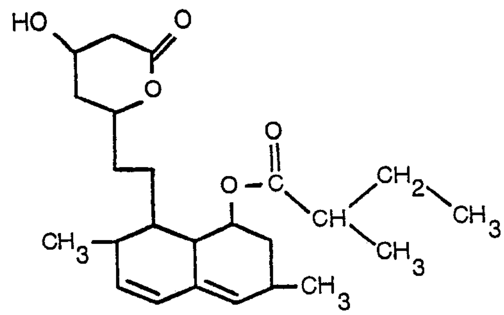

or

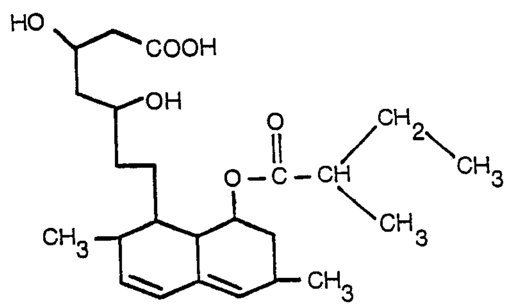

$a_3$) a (+) trans 6-E-4-hydroxy tetrahydropyran-2-one of the structure:

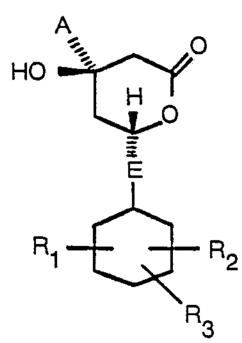

wherein

A is H or methyl;

E is a direct bond, $—CH_2—$, $—CH_2—CH_2—$, $—CH_2—CH_2—CH_2—$ or $—CH{=}CH—$;

$R_1$, $R_2$, and $R_3$ are each selected from

- H,
- halogen,
- $C_{1-4}$ alkyl,
- $C_{1-4}$ haloalkyl,
- phenyl,
- phenyl substituted by
  - halogen,
  - $C_{1-4}$ alkoxy
  - $C_{2-8}$ alkanoyloxy
  - $C_{1-4}$ alkyl,

$OR_4$ in which $R_4$ is
H,
$C_{2-8}$ alkanoyl,
benzoyl,
phenyl,
phenyl $C_{1-3}$ alkyl,
cinnamyl,
$C_{1-4}$ haloalkyl,
alkyl, preferably $C_{1-9}$ alkyl,
$C_{3-6}$ cyclo alkyl-$C_{1-3}$-alkyl,
adamantyl, adamantyl-$C_{1-3}$-alkyl or
substituted phenyl $C_{1-3}$ alkyl in each of which the substituents are selected from halogen,
$C_{1-4}$ alkoxy,
$C_{1-4}$ alkyl,
or
$C_{1-4}$ haloalkyl;

$a_4$) the corresponding hydroxy acids of the compounds of $a_1$) or $a_3$) in which the lactone ring is hydrolytically opened;
$a_5$) the pharmaceutically acceptable salts of said acids, and
$a_6$) the lower alkyl and phenyl dimethylamino or acetamino lower alkyl esters of said acids; and

b) a pharmaceutically acceptable nontoxic basic anion exchange resin capable of binding bile acids in a non-reabsorbable form in the gastrointestinal tract which is selected from

$b_1$) styrene-divinyl-benzene copolymers which contain quaternary ammonium functional groups, the main constituent of which is polystyrene,
$b_2$) water-insoluble high molecular weight copolymers obtained from polyalkylene polyamines and epichlorohydrin, glycerol-1,3-dichlorohydrin, an aliphatic bisepoxy compound or an alpha, omega-alkylene glycol,
$b_3$) esters of crosslinked polysaccharides,
$b_4$) quaternary ammonium anion exchange resins having a linear backbone which is free from both branching and cross-linking, comprising quaternized nitrogen atoms linked to each other through trimethylene groups,
$b_5$) water soluble anion exchange resins with a molecular weight of greater than 1500 with a plurality of ionizable amines sufficiently numerous to make the equivalent weight based on titration of the amino groups less than 500, wherein the polymer skeleton must be inert to degradation by digestive enzymes, which meet the in vitro test of binding at least 40% of glycocholic acid,
said respective components being present in ratio between 1:0.08 and 1:15,000.

2. Process of claim 1 in which said anionic exchange resin is a styrene-divinyl-benzene copolymer which contains quaternary ammonium functional groups, the main constituent of which is polystyrene trimethylbenzylammonium with $Cl^-$ anion.

3. Process of claim 1 in which said anion exchange resin is a copolymer of diethylenetriamine and 1-chloro-2,3-epoxypropane.

4. Process of claim 2 in which the cholesterol synthesis inhibitor is

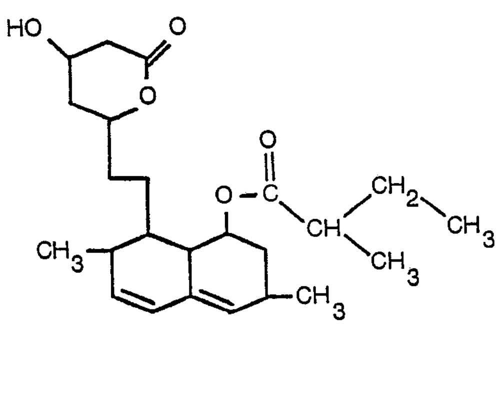

or

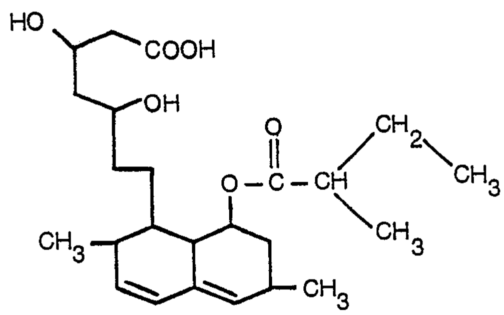

5. Process of claim 2 in which the cholesterol synthesis inhibitor is

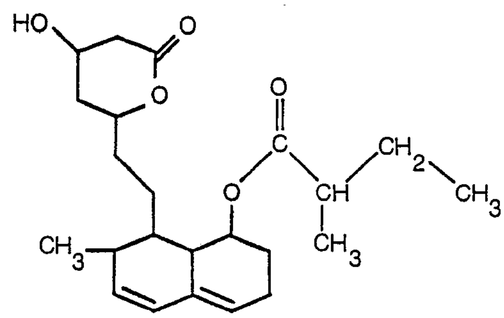

6. Process of claim 1 in which said anion exchange resin is poly[methyl-(3-trimethylammoniopropyl)imino-trimethylene-dihalide].

7. Process of claim 6 in which the cholesterol synthesis inhibitor is

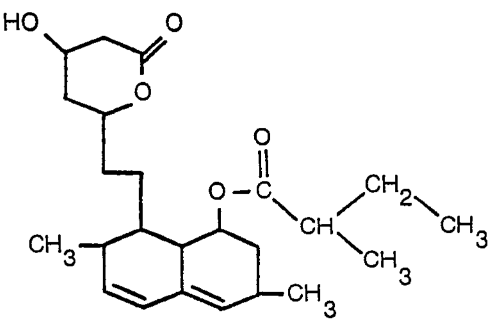

or

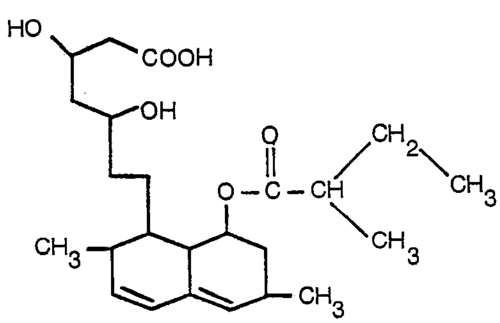

**Patentansprüche für die Vertragsstaaten: BE CH DE FR GB IT LU NL SE**

1. Eine pharmazeutische Zusammensetzung zur Verwendung bei der Behandlung von Hypercholesterämie, umfassend

a) einen Cholesterinsyntheseinhibitor, der auf den 3-Hydroxy-3-methylglutaryl-coenzym A-Reduktasespiegel mit einer zur Halbierung des Spiegels wirksamen Dosis ($ED_{50}$) von weniger als $10^{-6}$ einwirkt, der ausgewählt ist aus

$a_1$)

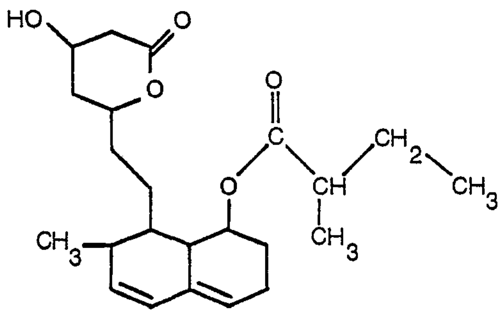

$a_2$)

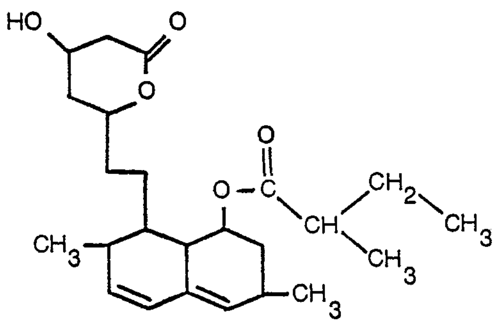

oder

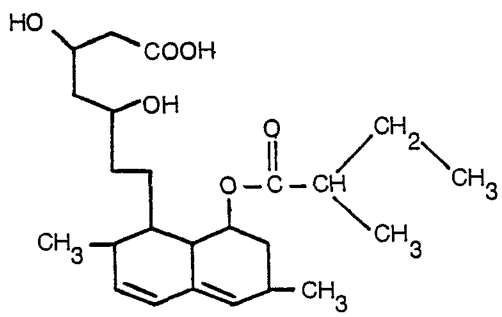

$a_3$) einem (+)-trans-6-E-4-Hydroxytetrahydropyran-2-on der Struktur:

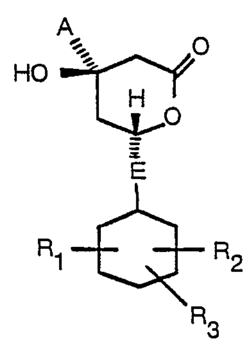

IV

worin

A Wasserstoff oder Methyl ist;
E eine direkte Bindung $—CH_2—$, $—CH_2—CH_2—$, $—CH_2—CH_2—CH_2—$ oder $—CH = CH—$ ist;
$R_1$, $R_2$ und $R_3$ jeweils aus
Wasserstoff,
Halogen,
$C_{1-4}$-Alkyl,
$C_{1-4}$-Halogenalkyl,
Phenyl,

Phenyl, das durch Halogen, $C_{1-4}$-Alkoxy, $C_{2-8}$-Alkanoyloxy, $C_{1-4}$-Alkyl substituiert ist, oder $OR_4$ ausgewählt sind, worin

$R_4$ Wasserstoff,
$C_{2-8}$-Alkanoyl,
Benzoyl,
Phenyl,
Halogenphenyl,
Phenyl-$C_{1-3}$-alkyl,
Cinnamyl,
$C_{1-4}$-Halogenalkyl,
Alkyl, vorzugsweise $C_{1-9}$-Alkyl,
$C_{3-6}$-Cycloalkyl-$C_{1-3}$-alkyl,
Adamantyl, Adamantyl-$C_{1-3}$-alkyl oder substituiertes Phenyl-$C_{1-3}$-alkyl ist, wobei in jedem davon die Substituenten aus
Halogen,
$C_{1-4}$-Alkoxy,
$C_{1-4}$-Alkyl oder
$C_{1-4}$-Halogenalkyl ausgewählt sind;

$a_4$) den entsprechenden Hydroxysäuren der Verbindungen $a_1$) oder $a_3$), in welchen der Lactonring hydrolytisch geöffnet ist;

$a_5$) den pharmazeutisch annehmbaren Salzen dieser Säuren, und

$a_6$) den Niedrigalkyl- und Phenyldimethylamino- oder Acetamino-niedrigalkylestern dieser Säuren; und

b) ein pharmazeutisch annehmbares, nicht-toxisches, basisches Anionenaustauscherharz, das zur Bindung von Gallensäuren in einer nicht-resorbierbaren Form im Gastrointestinaltrakt befähigt ist, welches ausgewählt ist aus

$b_1$) Styrol-divinyl-benzol-copolymeren, welche funktionelle quaternäre Ammoniumgruppen enthalten, deren Hauptbestandteil Polystyrol ist,

$b_2$) Wasserunlöslichen Copolymeren mit hohem Molekulargewicht, erhalten aus Polyalkylenpolyaminen und Epichlorhydrin, Glycerin-1,3-dichlorhydrin, einer aliphatischen Bisepoxyverbindung oder einem Alpha, omega-alkylenglycol,

$b_3$) Estern vernetzter Polysaccharide,

$b_4$) quaternären Ammoniumanionenaustauscherharzen, die ein lineares Gerüst aufweisen, welches sowohl von Verzweigung als auch von Vernetzung frei ist, enthaltend quaternisierte Stickstoffatome, die aneinander über Trimethylengruppen verbunden sind,

$b_5$) wasserlöslichen Anionenaustauscherharzen mit einem Molekulargewicht von mehr als 1500 mit einer Mehrzahl ionisierbarer Amine genügender Zahl, um das Äquivalentgewicht auf Basis der Titration der Aminogruppen kleiner als 500 zu machen, worin das Polymerskelett gegenüber einem Abbau durch Verdauungsenzyme inert sein muß,

welches dem in vitro-Test der Bindung hinsichtlich wenigstens 40% Glykocholsäure entspricht, wobei die betreffenden Komponenten in einem Verhältnis zwischen 1:0,08 und 1:15.000 vermischt sind.

2. Eine Zusammensetzung des Anspruchs 1, in welcher das anionische Austauscherharz ein Styrol-divinyl-benzol-copolymer ist, das funktionelle quaternäre Ammoniumgruppen enthält, deren Hauptbestandteil Polystyroltrimethylbenzylammonium mit $Cl^-$-Anion ist.

3. Eine Zusammensetzung des Anspruchs 1, in welcher das Anionenaustauscherharz ein Copolymer von Diethylentriamin und 1-Chlor-2,3-epoxypropan ist.

4. Die Zusammensetzung des Anspruchs 2, in welcher der Cholesterinsyntheseinhibitor

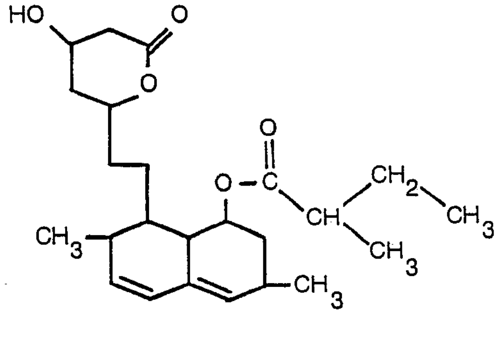

oder

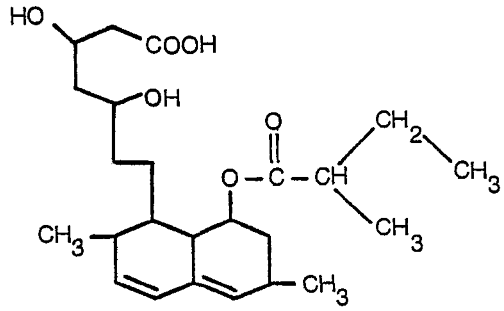

ist.

5. Die Zusammensetzung des Anspruchs 2, in welcher der Cholesterinsyntheseinhibitor

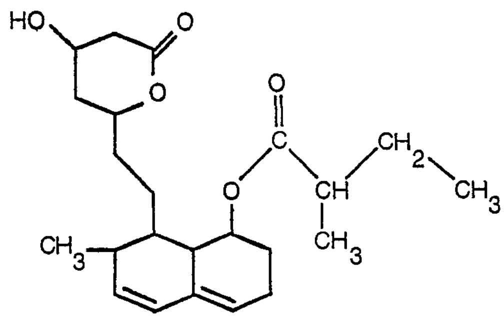

ist.

6. Die Zusammensetzung des Anspruchs 1, in welcher das Anionenaustauscherharz Poly[methyl-(3-trimethylammoniopropyl)iminotrimethylen-dihalogenid] ist.

7. Die Zusammensetzung des Anspruchs 6, in welcher der Cholesterinsyntheseinhibitor

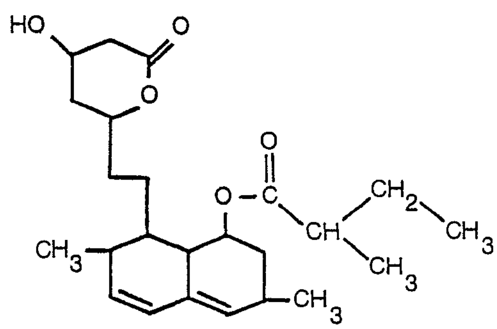

oder

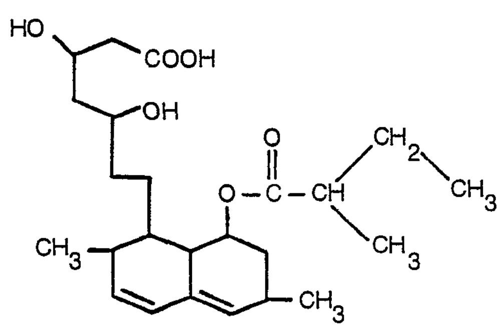

ist.

**Patentansprüche für den Vertragsstaat: AT**

1. Verfahren zur Herstellung einer pharmazeutischen Zusammensetzung zur Verwendung bei der Behandlung von Hypercholesterämie, wobei man

a) einen Cholesterinsyntheseinhibitor, der auf den 3-Hydroxy-3-methylglutaryl-coenzym A-Reduktasespiegel mit einer zur Halbierung des Spiegels wirksamen Dosis ($ED_{50}$) von weniger als $10^{-6}$ einwirkt, der ausgewählt ist aus

$a_1$)

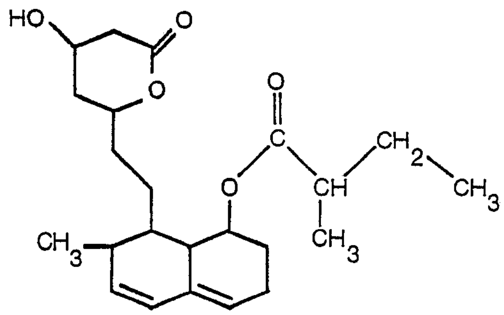

$a_2$)

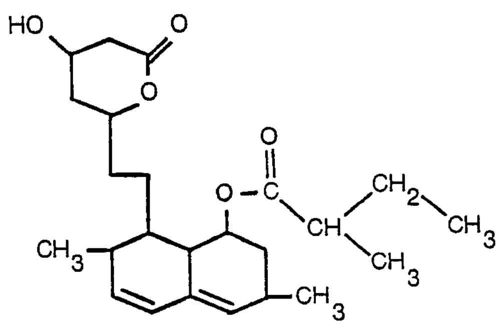

oder

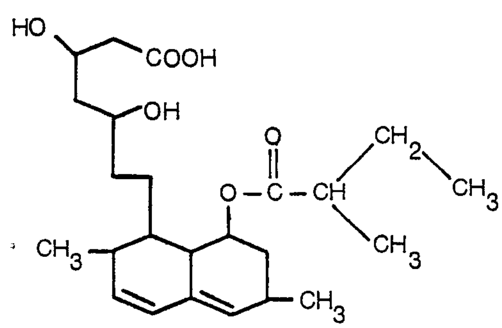

$a_3$) einem (+)-trans-6-E-4-Hydroxytetrahydropyran-2-on der Struktur:

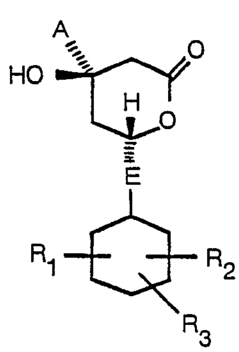

IV

worin

A Wasserstoff oder Methyl ist;

E eine direkte Bindung —$CH_2$—, —$CH_2$—$CH_2$—, —$CH_2$—$CH_2$—$CH_2$— oder —CH = CH— ist;

$R_1$, $R_2$ und $R_3$ jeweils aus

Wasserstoff,
Halogen,
$C_{1-4}$-Alkyl,
$C_{1-4}$-Halogenalkyl,
Phenyl,
Phenyl, das durch Halogen, $C_{1-4}$-Alkoxy, $C_{2-8}$-Alkanoyloxy, $C_{1-4}$-Alkyl substituiert ist, oder $OR_4$ ausgewählt sind, worin
$R_4$ Wasserstoff,
$C_{2-8}$-Alkanoyl,
Benzoyl,
Phenyl,
Halogenphenyl,
Phenyl-$C_{1-3}$-alkyl,
Cinnamyl,
$C_{1-4}$-Halogenalkyl,
Alkyl, vorzugsweise $C_{1-9}$-Alkyl,
$C_{3-6}$-Cycloalkyl-$C_{1-3}$-alkyl,
Adamantyl, Adamantyl-$C_{1-3}$-alkyl oder
substituiertes Phenyl-$C_{1-3}$-alkyl ist, wobei in jedem davon die Substituenten aus
Halogen,
$C_{1-4}$-Alkoxy,
$C_{1-4}$-Alkyl oder
$C_{1-4}$-Halogenalkyl ausgewählt sind;

$a_4$) den entsprechenden Hydroxysäuren der Verbindungen $a_1$) oder $a_3$), in welchen der Lactonring hydrolytisch geöffnet ist;
$a_5$) den pharmazeutisch annehmbaren Salzen dieser Säuren, und
$a_6$) den Niedrigalkyl- und Phenyldimethylamino- oder Acetamino-niedrigalkylestern dieser Säuren; und

b) ein pharmazeutisch annehmbares, nicht-toxisches, basisches Anionenaustauscherharz, das zur Bindung von Gallensäuren in einer nicht-resorbierbaren Form im Gastrointestinaltrakt befähigt ist, welches ausgewählt ist aus

$b_1$) Styrol-divinyl-benzol-copolymeren, welche funktionelle quaternäre Ammoniumgruppen enthalten, deren Hauptbestandteil Polystyrol ist.
$b_2$) wasserrunlöslichen Copolymeren mit hohem Molekulargewicht, erhalten aus Polyalkylenpolyaminen und Epichlorhydrin, Glycerin-1,3-dichlorhydrin, einer aliphatischen Bisepoxyverbindung oder einem Alpha, omega-alkylenglycol,
$b_3$) Estern vernetzter Polysaccharide,
$b_4$) quaternären Ammoniumanionenaustauscherharzen, die ein lineares Gerüst aufweisen, welches sowohl von Verzweigung als auch von Vernetzung frei ist, enthaltend quaternisierte Stickstoffatome, die aneinander über Trimethylengruppen verbunden sind,
$b_5$) wasserlöslichen Anionenaustauscherharzen mit einem Molekulargewicht von mehr als 1500 mit einer Mehrzahl ionisierbarer Amine genügender Zahl, um das Äquivalentgewicht auf Basis der Titration der Aminogruppen kleiner als 500 zu machen, worin das Polymerskelett gegenüber einem Abbau durch Verdauungsenzyme inert sein muß,
welches dem in vitro-Test der Bindung hinsichtlich wenigstens 40% Glykocholsäure entspricht, in einem Verhältnis zwischen 1:0,08 und 1:15.000 vermischt.

2. Verfahren nach Anspruch 1, in welchem das anionische Austauscherharz ein Styrol-divinylbenzol-copolymer ist, das funktionelle quaternäre Ammoniumgruppen enthält, deren Hauptbestandteil Polystyroltrimethylbenzylammonium mit $Cl^-$-Anion ist.

3. Verfahren nach Anspruch 1, in welchem das Anionenaustauscherharz ein Copolymer von Diethylentriamin und 1-Chlor-2,3-epoxypropan ist.

4. Verfahren nach Anspruch 2, in welches der Cholesterinsyntheseinhibitor

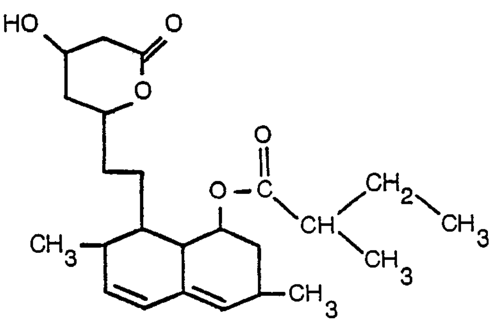

oder

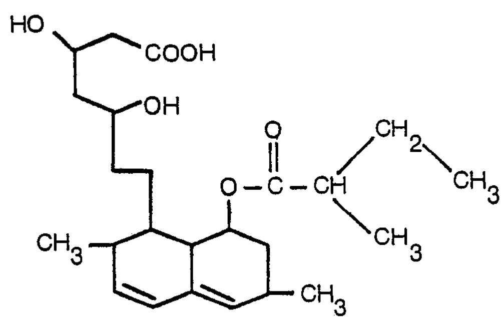

ist.

5. Verfahren nach Anspruch 2, in welchem der Cholesterinsyntheseinhibitor

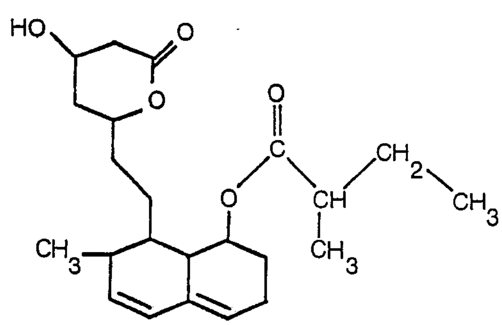

ist.

6. Verfahren nach Anspruch 1, in welchem das Anionenaustauscherharz Poly[methyl-(3-trimethylammoniopropyl)iminotrimethylen-dihalogenid] ist.

7. Verfahren nach Anspruch 6, in welchem der Cholesterinsyntheseinhibitor

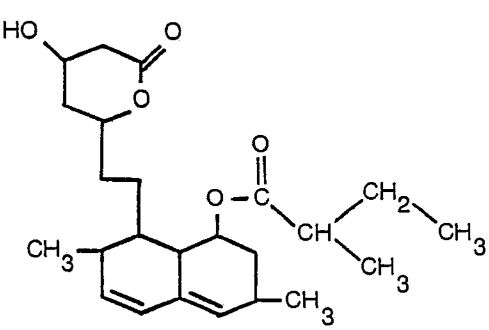

oder

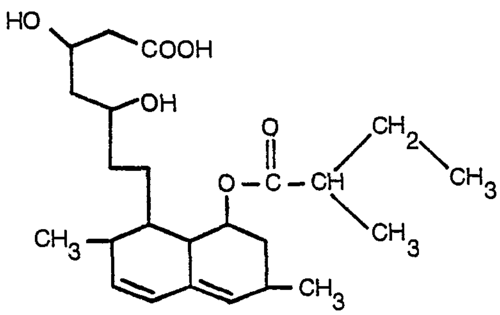

ist.

**Revendications pour les etats contractants: BE CH DE FR GB IT LU NL SE**

1. Une composition pharmaceutique utilisable pour le traitement d'hypercholestérémie, comprenant

a) un inhibiteur de synthèse de cholestérol agissant au niveau de la 3-hydroxy-3-méthylglutaryl-coenzyme A réductase avec une dose efficace moitié ($ED_{50}$) inférieure à $10^{-6}$ qui est choisi parmi

$a_1$)

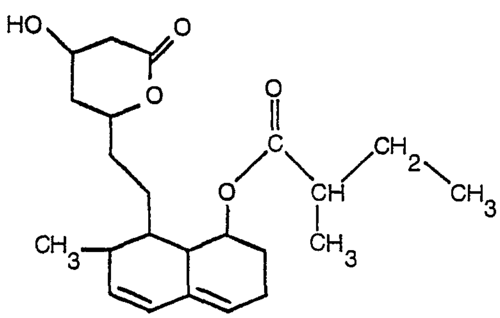

$a_2$)

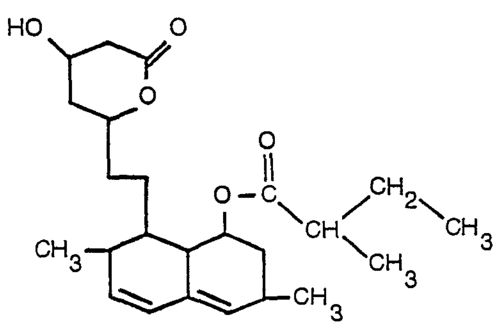

ou

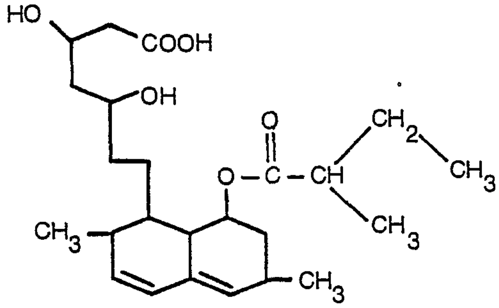

$a_3$) une (+)trans 6-E-4-hydroxytétrahydropyran-2-one de structure

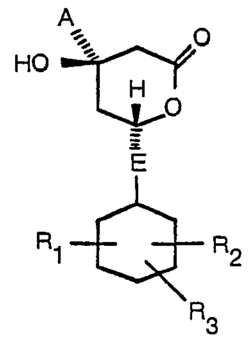

IV

où

A est H ou un méthyle;
E est une liaison directe, $—CH_2—$, $—CH_2—CH_2—$, $—CH_2—CH_2—CH_2—$ ou $—CH = CH—$,
$R_1$, $R_2$ et $R_3$ sont chacun choisis parmi
- H,
- un halogène,
- un $C_{1-4}$ alkyle,
- un $C_{1-4}$ haloalkyle,
- un phényle,
- un phényle substitué par
  - un halogène,
  - un $C_{1-4}$ alkoxy,
  - un $C_{2-8}$ alkanoyloxy,
  - un $C_{1-4}$ alkyle,

$OR_4$ dans lequel $R_4$ est
- H,
- un $C_{2-8}$ alkanoyle,
- un benzoyle,
- un phényle,
- un phényl $C_{1-3}$ alkyle,
- un cinnamyle,
- un $C_{1-4}$ haloalkyle,
- un alkyle, de préférence un $C_{1-9}$ alkyle,
- un $C_{3-6}$ cycloalkyl-$C_{1-3}$-alkyle,
- un adamantyle, adamantyl-$C_{1-3}$-alkyle, ou
- un phényl $C_{1-3}$ alkyle substitué dans chacun desquels les substituants sont choisis parmi
  - un halogène,
  - un $C_{1-4}$ alkoxy,
  - un $C_{1-4}$ alkyle,
  - ou un $C_{1-4}$ haloalkyle;

$a_4$) les hydroxy acides correspondants des composés de $a_1$) ou $a_3$) dans lesquels le cycle lactone est ouvert hydrolytiquement;

$a_5$) les sels acceptables d'un point de vue pharmaceutique desdits acides, et

$a_6$) les alkyl inférieur et phényldiméthylamino ou acétaminoalkyl inférieur esters desdits acides; et

b) une résine échangeuse d'anion, acceptable pharmaceutiquement, non toxique, basique capable de lier des acides de la bile sous une forme non réabsorbable dans la voie gastro-intestinale qui est choisie parmi

$b_1$) des copolymères styrène-divinyl-benzène qui contiennent des groupes fonctionnels ammonium quaternaire, dont le principal constituant est le polystyrène,

$b_2$) des copolymères à poids moléculaire élevé insolubles dans l'eau obtenus à partir de polyalkylène polyamines et d'épichlorohydrine, de glycérol-1,3-dichlorohydrine, un composé bisépoxy aliphatique ou un alpha, oméga-alkylène glycol,

$b_3$) des esters de polysaccharides à chaînes reliées,

$b_4$) des résines échangeuses d'anion ammonium quaternaire ayant une ossature linéaire qui est libre de structure branchée et de liaison entre chaîne, comprenant des atomes d'azote quaternisés liés les uns aux autres par des groupes triméthylène,

$b_5$) des résines échangeuses d'anion solubles dans l'eau avec un poids moléculaire supérieur à 1500 avec des amines en nombre suffisant pour rendre le poids équivalent basé sur une titration des

groupes amino inférieur à 500, où le squelette du polymère doit être inerte à une dégradation par des enzymes digestives, qui satisfait au test in vitro en liant au moins 40% d'acide glycocholique, lesdits composants respectifs étant mélangés dans un rapport compris entre 1/0,08 et 1/15.000.

2. Une composition selon la revendication 1 dans laquelle ladite résine échangeuse anionique est un copolymère styrène-divinyl-benzène qui contient des groupes fonctionnels ammonium quaternaire, dont le principal constituant est le polystyrène triméthylbenzylammonium avec un anion $Cl^-$.

3. Une composition selon la revendication 1 dans laquelle ladite résine échangeuse d'anion est un copolymère de diéthylènetriamine et de 1-chloro-2,3-époxypropane.

4. La composition de la revendication 2 dans laquelle l'inhibiteur de synthèse de cholestérol est

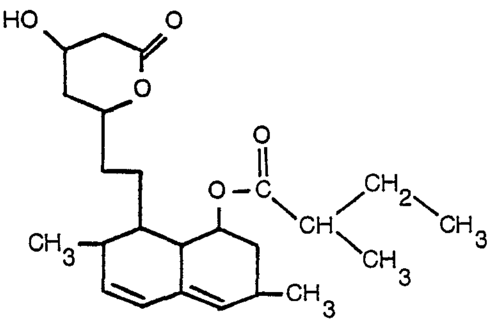

ou

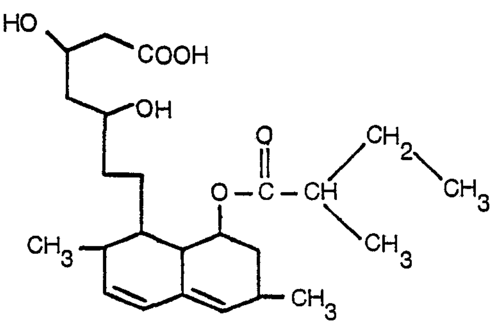

5. La composition de la revendication 2 dans laquelle l'inhibiteur de synthèse de cholestérol est

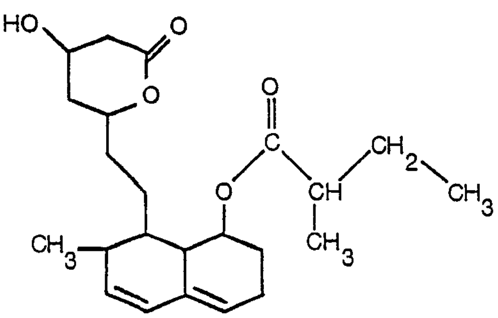

6. La composition de la revendication 1 dans laquelle la résine échangeuse d'anion est le poly[dihalogénure de méthyl-(3-triméthylammoniopropyl)iminotriméthylène].

7. La composition de la revendication 6 dans laquelle l'inhibiteur de synthèse de cholestérol est

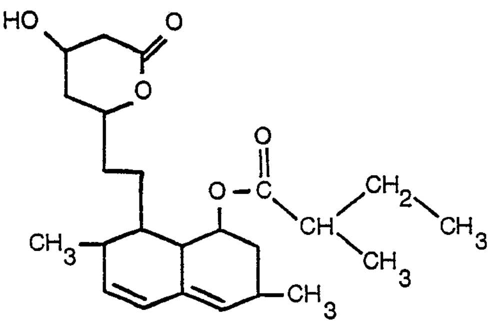

ou

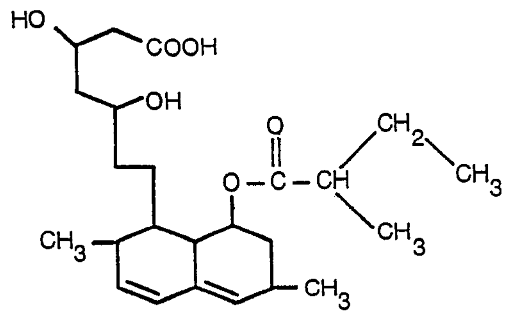

**Revendications pour l'etat contractant: AT**

1. Procédé de préparation d'une composition pharmaceutique utilisable pour le traitement d'hypercholestérémie, par mélange de

a) un inhibiteur de synthèse de cholestérol agissant à un niveau de 3-hydroxy-3-méthylglutaryl-coenzyme A avec une dose efficace moitié ($ED_{50}$) inférieure à $10^{-6}$ qui est choisi parmi

$a_1$)

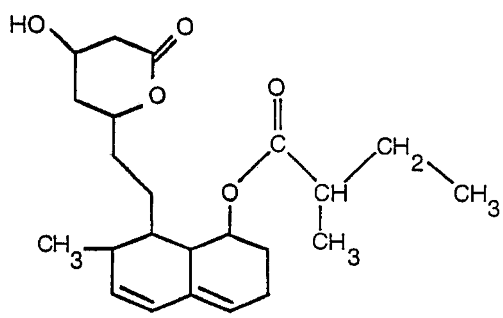

$a_2$)

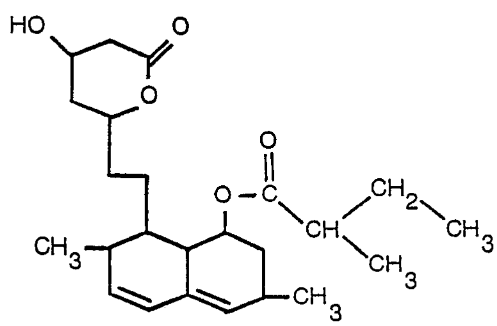

ou

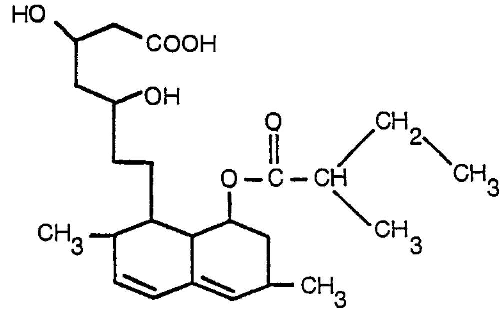

$a_3$) un (+) trans 6-E-4-hydroxytétrahydropyran-2-one de structure

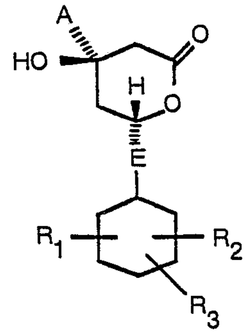

où

A est H ou un méthyle;
E est une liaison directe, $—CH_2—$, $—CH_2—CH_2—$, $—CH_2—CH_2—CH_2—$ ou $—CH = CH—$;
$R_1$, $R_2$ et $R_3$ sont chacun choisis parmi H,
un halogène,
un $C_{1-4}$ alkyle,
un $C_{1-4}$ haloalkyle,
un phényle,
un phényle substitué par
un halogène,
un $C_{1-4}$ alkoxy,
un $C_{2-8}$ alkanoyloxy,
un $C_{1-4}$ alkyle,
$OR_4$ dans lequel $R_4$ est
un H,
un $C_{2-8}$ alkanoyle,
un benzoyle,
un phényle,
un phényl $C_{1-3}$ alkyle,
un cinnamyle,
un $C_{1-4}$ haloalkyle,
un alkyle, de préférence un $C_{1-9}$ alkyle,
un $C_{3-6}$ cycloalkyl-$C_{1-3}$ alkyle,
un adamantyle, adamantyl-$C_{1-3}$ alkyle,
ou
un phényl $C_{1-3}$ alkyle substitué dans chacun desquels les substituants sont choisis parmi
un $C_{1-4}$ alkoxy,
un $C_{1-4}$ alkyle,
ou
un $C_{1-4}$ haloalkyle;

$a_4$) les hydroxy acides correspondants des composés de $a_1$) ou $a_3$) dans lesquels le cycle lactone est hydrolytiquement ouvert;

$a_5$) les sels acceptables d'un point de vue pharmaceutique desdits acides, et

$a_6$) les alkyl inférieur et phényldiméthylamino ou acétaminoalkyl inférieur esters desdits acides; et

b) une résine échangeuse d'anion, acceptable pharmaceutiquement, non toxique, basique, capable de lier des acides de la bile sous une forme non réabsorbable dans la voie gastro-intestinale qui est choisie parmi

$b_1$) des copolymères styrène-divinyl-benzène qui contiennent des groupes fonctionnels ammonium quaternaire, dont le principal constituant est le polystyrène,

$b_2$) des copolymères à poids moléculaire élevé, insolubles dans l'eau obtenus à partir de polyalkylène polyamines et d'épichlorohydrine, de glycérol-1,3-dichlorohydrine, un composé bisépoxy aliphatique ou un alpha, oméga-alkylène glycol,

$b_3$) des esters de polysaccharides à chaînes reliées,

$b_4$) des résines échangeuses d'anion ammonium quaternaire ayant une ossature linéaire qui est libre de structure branchée et de liaison entre chaîne, comprenant des atomes d'azote quaternisés liés les uns aux autres par des groupes méthylène,

$b_5$) des résines échangeuses d'anion solubles dans l'eau avec un poids moléculaire supérieur à 1500 avec des amines ionisables en nombre suffisant pour rendre le poids équivalent basé sur une

titration des groupes amino inférieur à 500, où le squelette du polymère doit être inerte à une dégradation par des enzymes digestives, qui satisfait au test in vitro en liant au moins 40% d'acide glycocholique,
lesdits composants respectifs étant présents dans un rapport compris entre 1:0.08 et 1:15.000.

2. Procédé de la revendication 1 dans lequel ladite résine échangeuse anionique est un copolymère styrène-divinyl-benzène qui contient des groupes fonctionnels ammonium quaternaire, dont le principal constituant est le polystyrène triméthylbenzylammonium avec un anion $Cl^-$.

3. Procédé de la revendication 1 dans lequel ladite résine échangeuse d'anion est un copolymère de diéthylènetriamine et de 1-chloro-2,3-époxypropane.

4. Procédé de la revendication 2 dans lequel l'inhibiteur de synthèse de cholestérol est

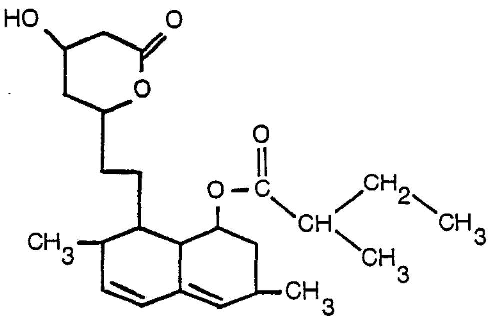

ou

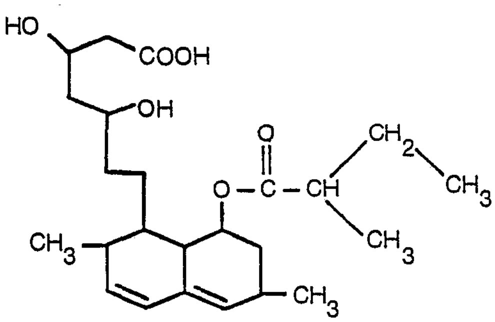

5. Procédé de la revendication 2 dans lequel l'inhibiteur de synthèse de cholestérol est

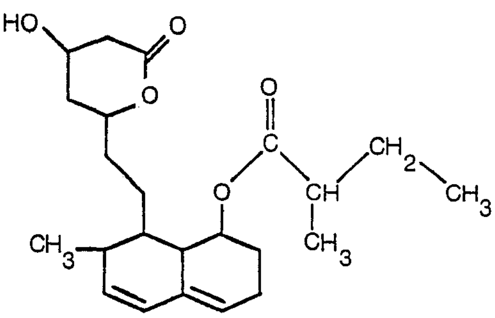

6. Procédé de la revendication 1 dans lequel ladite résine échangeuse d'anion est le poly[méthyl(3-triméthylammoniopropyl)iminotriméthylène-dihalogénure].

7. Procédé de la revendication 6 dans lequel l'inhibiteur de synthèse de cholestérol est

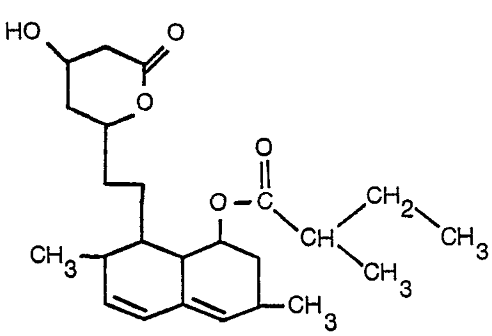

ou

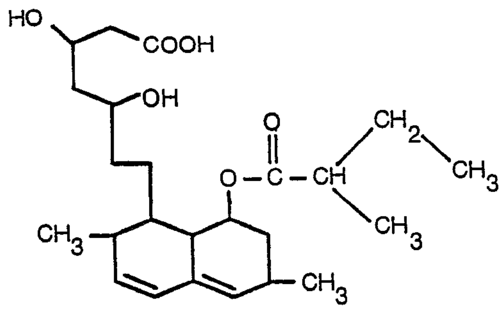

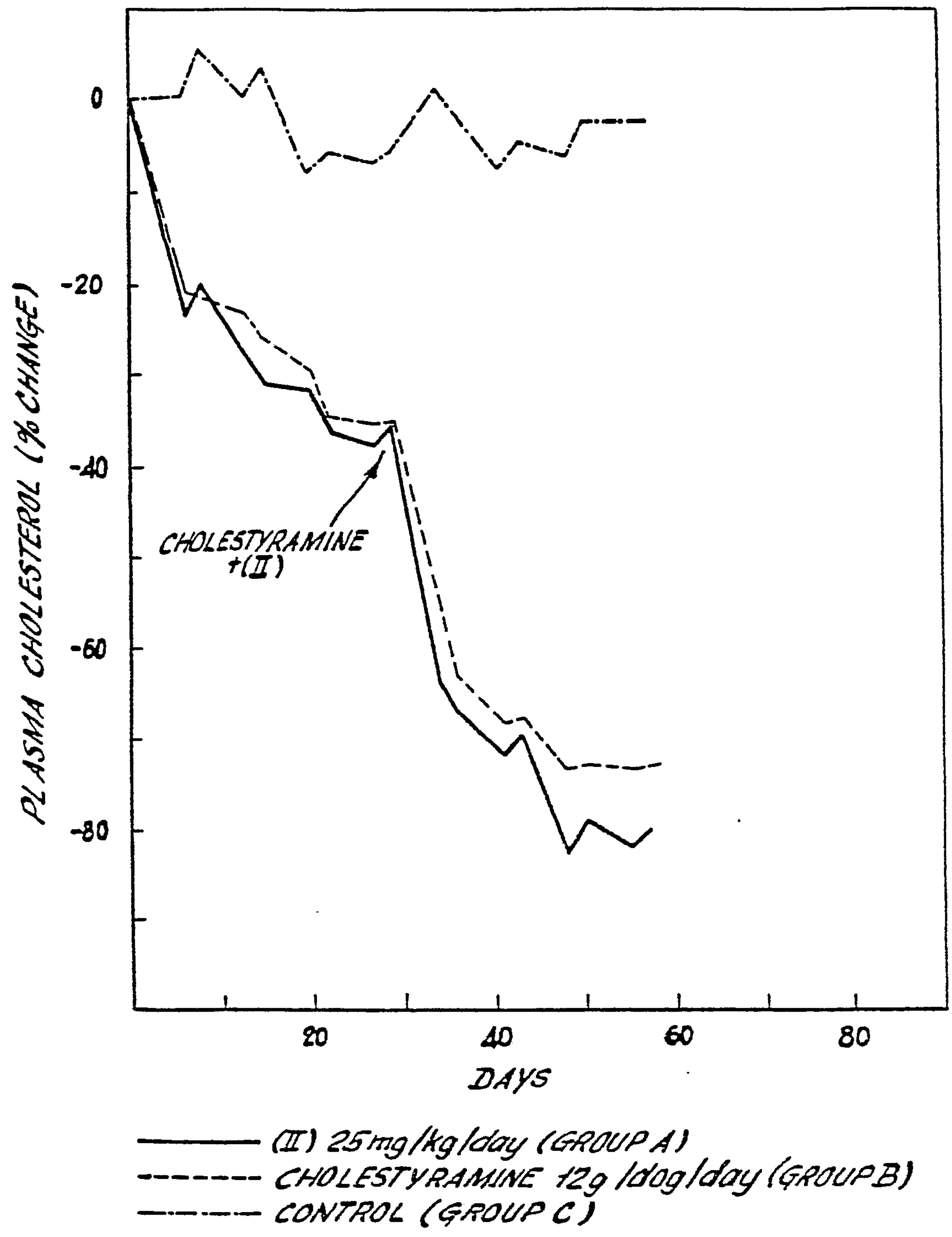
0
-20
-40
-60
-80
PLASMA CHOLESTEROL (% CHANGE)
CHOLESTYRAMINE
+(II)
20
40
60
80
DAYS
(II) 25mg/kg/day (GROUP A)
CHOLESTYRAMINE 12g /dog/day (GROUP B)
CONTROL (GROUP C)

Fig.1